# EUROPEAN PATENT SPECIFICATION

(11) **EP 2 435 019 B1**
(45) Date of publication and mention of the grant of the patent: **23.09.2015**
(21) Application number: 10723532.7
(22) Date of filing: 31.05.2010
(51) Int. Cl.: A61K 8/42, A61K 31/16, C07C 233/09, C07C 233/13, A61K 9/00, A61K 8/49, A61Q 7/02, C07D 333/20, C07D 237/20, C07D 277/82, C07D 213/64, C07D 307/52, C07D 239/42, A61Q 19/00, C07C 233/20, C07D 277/46, C07D 213/40, C07D 257/04, C07D 277/10

(54) **DODECA-2E,4E-DIENE AMIDES AND THEIR USE AS MEDICAMENTS AND COSMETICS**
DODECA-2E,4E-DIENAMIDE, DEREN HERSTELLUNG UND DEREN VERWENDUNG ALS ARZNEIMITTEL UND KOSMETIKA
AMIDES DU DODECA-2E,4E-DIENE, LEUR PREPARATION ET UTILISATION EN TANT QUE MEDICAMENTS ET PRODUITS COSMETIQUES

(30) Priority: 29.05.2009 EP 09007245
(43) Date of publication of application: 04.04.2012
(73) Proprietor: Dr. August Wolff GmbH & Co. KG Arzneimittel, 33611 Bielefeld (DE)
(72) Inventor: GERTSCH, Jürg, CH-3012 Bern (CH)
(74) Representative: Ter Meer Steinmeister & Partner
(86) International application number: PCT/EP2010/003299
(87) International publication number: WO 2010/136221

(56) References cited:
- WO-A-2004/000787
- WO-A-2006/002493
- WO-A-2009/041787
- WO-A-2009/065090
- US-A- 4 692 510
- US-A- 4 742 047
- WOELKART KARIN ET AL: "The endocannabinoid system as a target for alkamides from Echinacea angustifolia roots" PLANTA MEDICA, THIEME, STUTTGART, DE, vol. 71, no. 8, 1 August 2005 (2005-08-01) , pages 701-705, XP002489201 ISSN: 0032-0943 [retrieved on 2005-08-11]
- JÜRG GERTSCH: "Immunomodulatory Lipids in Plants: Plant Fatty Acid Amides and the Human Endocannabinoid System" PLANTA MEDICA, THIEME, STUTTGART, DE, vol. 74, 1 January 2008 (2008-01-01), pages 638-650, XP007909743 ISSN: 0032-0943
- LALONE CARLIE A ET AL: "Echinacea species and alkamides inhibit prostaglandin E-2 production in RAW264.7 mouse macrophage cell" JOURNAL OF AGRICULTURAL AND FOOD CHEMISTRY, AMERICAN CHEMICAL SOCIETY, WASHINGTON, US, vol. 55, no. 18, 5 September 2007 (2007-09-05), pages 7314-7322, XP007909737 ISSN: 0021-8561
- DATABASE EMBASE [Online] ELSEVIER SCIENCE PUBLISHERS, AMSTERDAM, NL; 2008, SKOPINSKA-ROZEWSKA E ET AL: "Aqueous and hydro-alcoholic extracts of Echinacea purpurea (L) Moench as traditional herbal remedies with immunotropic activity" XP002590488 Database accession no. EMB-2008266046 & CENTRAL-EUROPEAN JOURNAL OF IMMUNOLOGY 2008 PL, vol. 33, no. 2, 2008, pages 78-82, ISSN: 1426-3912
- STOEHR J R ET AL: "CONSTITUENTS OF CHINESE PIPER SPECIES AND THEIR INHIBITORY ACTIVITY ON PROSTAGLANDIN AND LEUKOTRIENE BIOSYNTHESIS IN VITRO" JOURNAL OF ETHNOPHARMACOLOGY, ELSEVIER SCIENTIFIC PUBLISHERS LTD, IE, vol. 75, no. 2/03, 1 January 2001 (2001-01-01), pages 133-139, XP001122249 ISSN: 0378-8741
- SIDDIQUI BINA SHAHEEN ET AL: "New insecticidal amides from petroleum ether extract of dried Piper nigrum L. whole fruits" CHEMICAL AND PHARMACEUTICAL BULLETIN, PHARMACEUTICAL SOCIETY OF JAPAN, TOKYO, JP, vol. 52, no. 11, 1 November 2004 (2004-11-01), pages 1349-1352, XP007909749 ISSN: 0009-2363
- MATTHIAS ANITA ET AL: "Bioavailability of Echinacea constituents: Caco-2 monolayers and pharmacokinetics of the alkylamides and caffeic acid conjugates." MOLECULES (BASEL, SWITZERLAND) 2005 LNKD- PUBMED:18007516, vol. 10, no. 10, 2005, pages 1242-1251, XP007913748 ISSN: 1420-3049
- ABARBRI M ET AL: "A synthetic approach to natural dienamides of insecticidal interest" SYNTHETIC COMMUNICATIONS, TAYLOR & FRANCIS GROUP, PHILADELPHIA, PA, vol. 28, no. 2, 1 January 1998 (1998-01-01), pages 239-249, XP009122510 ISSN: 0039-7911
- ADESINA S K ET AL: "GC/MS investigations of the minor constituents of Piper guineense stem" PHARMAZIE, DIE, GOVI VERLAG, ESCHBORN, DE, vol. 57, no. 9, 1 September 2002 (2002-09-01), pages 622-627, XP001539548 ISSN: 0031-7144
- OMAFUVBE BO ET AL: "Quality Assurance of Stored Pepper (Piper guineense) Using Controlled Processing Methods" PAKISTAN JOURNAL OF NUTRITION, vol. 3, no. 4, 2004, pages 244-249, XP002547099
- CHICCA A ET AL: "Synergistic immunomopharmacological effects of N-alkylamides in Echinacea purpurea herbal extracts" INTERNATIONAL IMMUNOPHARMACOLOGY, ELSEVIER, AMSTERDAM, NL, vol. 9, no. 7-8, 1 July 2009 (2009-07-01), pages 850-858, XP026159351 ISSN: 1567-5769 [retrieved on 2009-03-19]

## Description

The present invention relates to a novel class of compounds, namely specific dodeca-*2E*,*4E-*diene amides and their use as a medicament, preferably as a dermatologic agent, and as cosmetics. These novel compounds target the endocannabinoid system and are particularly useful in treating and/or preventing inflammation, irritation, itching, pruritus, pain, oedema, and/or pro-allergic or allergic conditions in a patient. Usually they are topically applied to the skin or mucosa of a mammal in the form of a pharmaceutical or cosmetic composition comprising the compound and a pharmaceutically and/or cosmetically acceptable carrier.

### Background of the Invention

The endocannabinoid system (ECS) comprises cannabinoid receptors (CB₁ and CB₂, TRPV1 and potentially also GPR55), arachidonic acid-derived ligands, and their regulatory enzymes. The importance of the endocannabinoid system in peripheral tissues has been demonstrated in numerous recent studies (Di Marzo V. Targeting the endocannabinoid system: to enhance or reduce? Nat Rev Drug Discov. 2008 May; 7(5):438-55). Whereas the activation of the peripheral endocannabinoid system is often associated with anti-inflammatory and immunosuppressive effects, in the skin the role of the ECS is more complex. CB₂ receptor activation has been shown to trigger endorphins that locally act analgesic (Ibrahim MM, Porreca F, Lai J, Albrecht PJ, Rice FL, Khodorova A, Davar G, Makriyannis A, Vanderah TW, Mata HP, Malan TP Jr. CB2 cannabinoid receptor activation produces antinociception by stimulating peripheral release of endogenous opioids. Proc Natl Acad Sci U S A. 2005 Feb 22;102(8):3093-8). Studying CB knockout mice, it has been observed that the ECS and the CB₁ receptor can inhibit the pathogenesis of allergic contact dermatitis (Karsak M, Gaffal E, Date R, Wang-Eckhardt L, Rehnelt J, Petrosino S, Starowicz K, Steuder R, Schlicker E, Cravatt B, Mechoulam R, Buettner R, Werner S, Di Marzo V, Tüting T, Zimmer A. Attenuation of allergic contact dermatitis through the endocannabinoid system. Science. 2007 Jun 8;316(5830):1494-7). Interestingly, in this study it was shown that inhibitors of anandamide degradation via fatty acid amide hydrolase (FAAH) could be a promising therapeutic strategy to treat different forms of dermatitis. Overall, the endocannabinoid system in the skin is present, both CB₁ and CB₂ receptors have been detected in keratinocytes and fibroblasts and the endocannabinoids are released in the skin where they seem to regulate multiple signals involved in inflammation. Anandamide exerts potent anti-inflammatory and anti-allergic effects (Leonti M, Casu L, Raduner S, Cottiglia F, Floris C, Altmann KH, Gertsch J. Falcarinol is a covalent cannabinoid CB1 receptor antagonists and induces pro-allergic effects in skin. Biochem. Pharmacol. 2010, 79: 1815-1826 ). Moreover, anandamide reuptake inhibitors have been shown to exert a number of beneficial effects including neuropathic and peripheral pain (Yates M L, Baker EL. Inactivation and Biotransformation of the endogenous cannabinoids anadamide and 2-arachidonoyl glycerol. Mol. Pharmacol. 2009, 76: 11-17.). However, the mechanisms underlying are not yet fully understood and there still remain numerous questions to be answered.

The compound class of dodeca-*2E*,*4E*-diene amides is known in the prior art. WO09/065090 claims Glidobactin A (GibA), which consists of a twelve-membered ring linked via threonine to the dodeca-*2E,4E*-dienyl moiety, and analogs as proteasome inhibitors. In US4,692,510 the compound is claimed to have antibacterial, antifungal and antitumor activity. Glidobactin A (BU-2867T A) is used as starting material for the synthesis of semi-synthetic BU-2867T antibiotics (US4,742,047).

Chemical and enzymatic degradations and subsequent spectral analyses were used to elucidate the structures of antitumor antibiotics, Glidobactins A, B and C (Oka M. Glidobactins A, B and C, new antitumor antibiotics II. Structure elucidation. J. Antibiotics. 1988:41;1338-1350). *N-*[4(*S*)-Amino-2(*E*)-pentenoyl]-^{α}*N*-[*N-*2(*E*),4(*E*)-dodecandienoyl-L-threoninyl]-*erythro*-4-hydroxy-L-lysine was formed during mild acid hydrolysis of Glidobactin A. The compound is described to be completely bio-inactive. Enzymatic cleavage of GibA by papain yielded 2,4-dodecadienoylthreonine. In a second study the same group investigated the influence of chemical modifications on Glidobactin on its biological function (Oka M. Chemical Modification on the antitumor antibiotic Glidobactin. J. Antibiotics. 1988:41;1812-1822). Modifications of the fatty acid moiety were carried out, threonine was replaced with other amino acids and dihydro-derivatives of GibA were obtained using hydrogenated glidobamin as precursor. Selected compounds contain a dodeca-*2E,4E*-diene amide moiety. Data for antitumor activity of the compounds is provided.

Further, spicamycin is an antitumor antibiotic produced by *Streptomyces alanosinicus* 879-MT₃. Derivatives of spicamycin with modified fatty acid backbone have been synthesized and their antitumor activity was assessed (Sakai T. Structure-antitumor relationship of semisynthetic spicamycin derivatives. J. Antibiotics. 1995:48;1467-1480). 6-{4-Deoxy-4-[(2*E*,4*E*)-dodecadienoyl]glycylamino-L-*glycero*-β-L-*manno*-heptopyranosylamino}-9*H-*purine was found to show excellent antitumor activity with a therapeutic index of 16.

Analysis of extracts of *Piper guineense* stem by GC-MS resulted in the detection and identification of three different dodeca-*2E,4E*-diene amides, namely N-isobutyl-2,4-dodecadienamide, N-pyrrolidyl-2,4-dodecadienamide and N-piperidyl-2,4-dodecadienamide (Adesina S.K. GC/MS investigations of the minor constituents of Piper guineense stem. Pharmazie. 2002;57:622-627). Biological data for the compounds, however, is not provided.

Moreover, synthesis of N-isobutyl-dodeca-trans-2,trans-4-dienamide and N-(2'-p-hydroxyphenylethyl)-dodeca-trans-2,trans-4-dienamide starting from methyl dodeca-trans-2,trans-4-dienoate has been described (Burden R.S. Amides of vegetable origin. Part XII. A new series of alka-2,4-dienoic tyramine-amides from Anacyclus pyrethrum D.C. (Compositae). J. Chem. Soc. Sec. C: Org. Chem. 1969:2477-2481). The paper, however, does not disclose biological data for the compounds.

In addition, dodeca-*2E,4E*-diene isobutyl amide has been disclosed as part of an Echinacea formulation in WO06/002493. Further, the synthesis of dodeca-*2E,4E*-diene isobutyl amide has been described by using a modified Wittig reaction (Sharma S.D. Synthesis of N-isobutyl-2(E),4(E)-dienamides & N-isobutyldodeca-2(E),4(E)-dienamides. Indian J. Chem. 1979;18B:81-82) and via palladium-catalyzed cross coupling reactions (Abarbi M. Efficient synthesis of conjugated (2E)- or (2Z)-en-4-ynoic acids and (2E,4E)- or (2Z,4E)-dienoic acids via palladium-catalyzed cross coupling. Synthesis. 1996;1:82-86).

For alkamides isolated from the roots of *Echinacea angustifolia* affinity towards CB₁ and CB₂ receptors was investigated. In addition, inhibition of FAAH was also measured (Woelkart K. The endocannabinoid system as a target for alkamides from Echinacea angustifolia roots. Planta Med. 2005;71:701-705). Therein, dodeca-*2E,4E*-diene isobutyl amide was found to have affinities to both CB receptors in the single digit micromolar range and to be only a very weak inhibitor of FAAH.

In a perspective effects of plant fatty acid amides on the human endocannabinoid system resulting in immunomodulation, dodeca-*2E,4E*-diene isobutyl amide is described to have an affinity towards CB₂ receptors with Kᵢ <100 nM (Gertsch J. Immunomodulatory lipids in plants: Plant fatty acid amides and the human endocannabinoid system. Planta Med. 2008;74:638-650). In contrast to other compounds, dodeca-*2E,4E*-diene isobutyl amide did not inhibit FAAH. The compound is described to exhibit pronounced lipid polymorphism and to form micelles in aqueous medium. Self assembly of such "soap-like" bioactive compounds may not only influence pharmacodynamics, but also pharmacokinetics of other lipophilic compounds.

Inhibition of prostaglandin E₂ production may be one of processes contributing to the anti-inflammatory capabilities of *Echinacea* species (LaLone C.A. Echinacea species and alkamides inhibit prostaglandin E2 production in RAW264.7 mouse macrophage cells. J. Agric. Food Chem. 2007;55:7314-7322). Dodeca-*2E,4E*-diene isobutyl amide (Bauer alkamide 11) was inhibiting PGE₂ production at 50 µM. No significant cytotoxicity was observed at this concentration. However, this is contradicted by the finding of another group where said compound was found to cause 50% cell death at 50 µM (Chen Y. Macrophage activating effects of new alkamides from the roots of Echinacea species. J. Nat. Prod. 2005;68:773-776).

Furthermore, extracts from 19 *Piper* species have been screened for their 5-lipoxygenase (5-LOX) and cyclooxygenase-1 (COX-1) inhibitory potential (Stöhr J.R. Constituents of Chinese Piper species and their inhibitory activity on prostaglandin and leukotriene biosynthesis in vitro. J. Ethnopharmacol. 2001;75:133-139). Several extracts were able to considerably inhibit the biosynthesis of leukotriene (5-LOX) and/or prostaglandin (COX-1) at concentrations of 10 µg/ml and 26 µg/ml, respectively. Some of these extracts also contained dodeca-*2E*,*4E*-diene isobutyl amide.

Further, WO09/041787 discloses the use of a *Piper nigrum* extract for preventing and treating inflammatory or infectious diseases. Deca-*2E,4E*-diene isobutyl amide is described as one of the ingredients contained.

Synthesis of deca-*2E,4Z*-diene isobutyl amide (cis-Pellitorin) and it's use to give a prickly, "hot" taste to foods and oral hygiene products are disclosed in WO04/000787.

Dienamides of insecticidal interest can be synthesized using a Stille cross-coupling (Abarbri M. A Synthetic approach to natural dienamides of insecticidal interest. Synth. Commun. 1998;28:239-249). N-Isobutyl-*2E,4E*-dodecadienamide and N-pyrrolidyl-*2E,4E*-dodecadienamide are described as specific examples.

Two-carbon homologation to unbranched or methyl-branched unsaturated aldehydes using diethyl methylformylphosphonate dimethylhydrazone and diethyl ethylformyl-2-phosphonate dimethylhydrazone, containing a protected aldehyde group instead of the usual ester group has been described (Petroski R.J. New phosphonate reagents for aldehyde homologation. Synth. Commun. 2007;37:3841-3854). (*2E,4E*)-Dodeca-2,4-dienal is not mentioned as specific example.

As it becomes evident from the above, 2*E*,4*E*-dodecadiene amides, however, are not unambiguously disclosed in the prior art to be suitable and active in the treatment and prevention of skin disorders.

Further, even if a large number of dermatologic agents and preparations is known in the prior art for treating any kinds of skin discomfort, there is a still a strong demand to find new active agents being more effective, requiring even reduced amounts to be applied, and having less undesirable side-effects.

Thus, the object underlying the present invention is the provision of new compounds suitable as medicaments. It is a further object of the present invention to provide for new compounds suitable as dermatologic agents for treating and preventing several conditions including inflammation, irritation, itching, pruritus, pain, oedema, and/or pro-allergic or allergic conditions etc., particularly of the skin and mucosa of a mammal, which compounds are more effective, require reduced amounts of active ingredient as compared to prior art compounds and, and have less undesirable side-effects.

### Summary of the Invention

The objects as mentioned above have surprisingly been solved in accordance with the present invention. Thus, the present invention relates to a new class of compounds of specific dodeca-*2E,4E*-diene amides as defined in claims 1 and 12.

Moreover, the present invention relates to the use of these compounds as a medicament, preferably a dermatologic agent, and as a cosmetic. The compounds are effective in the treatment and/or prevention of inflammation, irritation, itching, pruritus, pain, oedema, and/or pro-allergic or allergic conditions, particularly of the skin and mucosa of a mammal. Further conditions include hair growth (e.g. forms of alopecia, effluvium) and sebaceous gland disorders (e.g. acne, seborrhea), benign and malignant skin tumors, hyperproliferative skin diseases (e.g. psoriasis), excessive hair growth (e.g. hirsutism), different forms of dermatitis, dry skin conditions, and systemic sclerosis (scleroderma).

### Description of the Figures

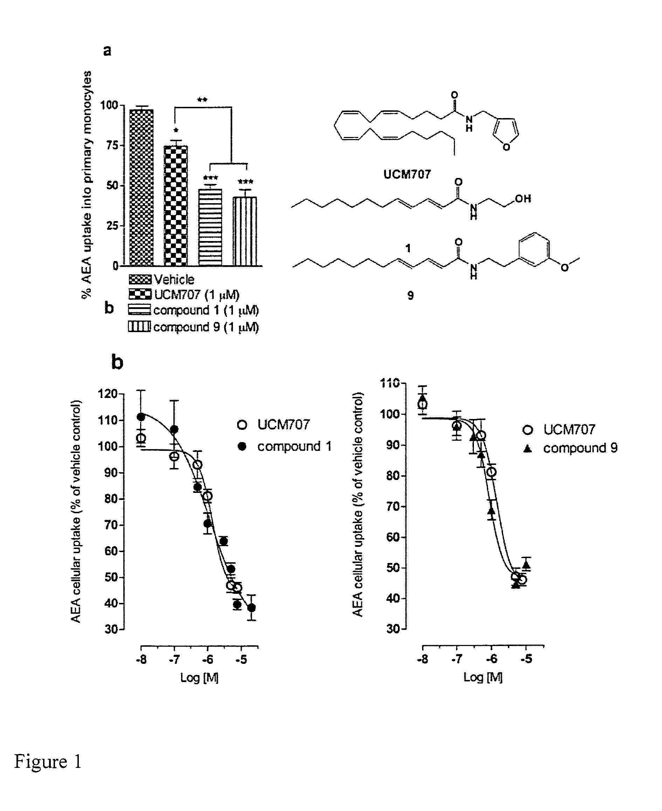

Figure 1: AEA re-uptake inhibition of 2*E*,4*E*-dieneamides **1** and **9** into human primary monocytes and U937 cells. (a) In human primary monocytes, 1 and 9 (1 µM) potently inhibited [³H]-AEA uptake while UCM707 (1 µM) was clearly less active. (b) The EC₅₀ value of the positive control UCM707 (1421 ± 296 nM) is in the same range as the EC₅₀ values for 1 (1227 ± 305 nM) and 9 (869 ± 172 nM). Data are mean values ± SEM of 3 independent experiments in a sigmoidal dose-response curve.

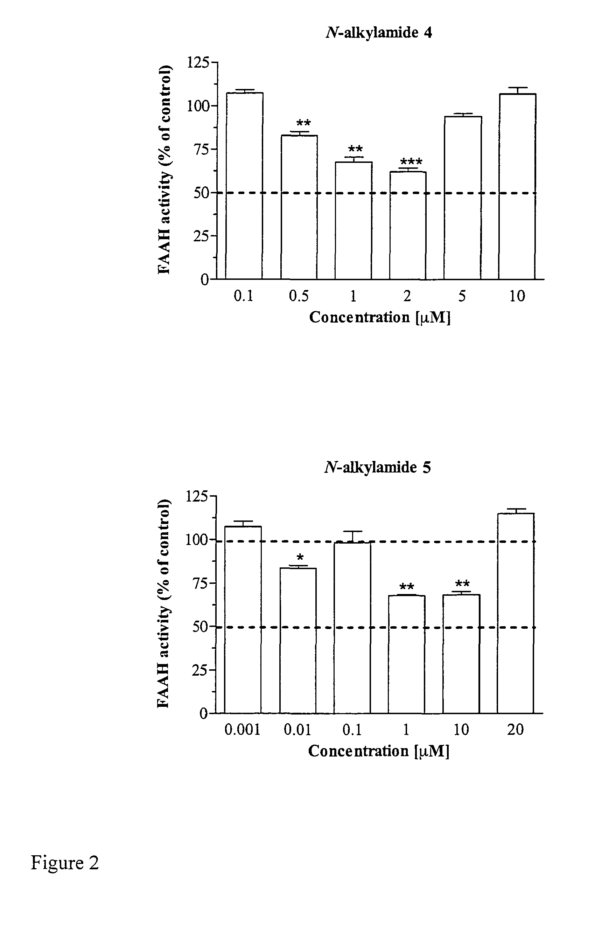

Figure 2: Concentration-dependent effects of **4** and **5** on FAAH activity. Compound **4** concentration-dependently inhibited FAAH activity until the CMC range (< 3 µM) with a max. inhibition of 40% at 2 µM. Compound **5** showed a multiphasic FAAH inhibition pattern, which may be related to the CMC (< 1 µM). The maximal inhibition (32%) of **5** was measured at 1 µM. Data are mean values ± SEM of 3 independent experiments.

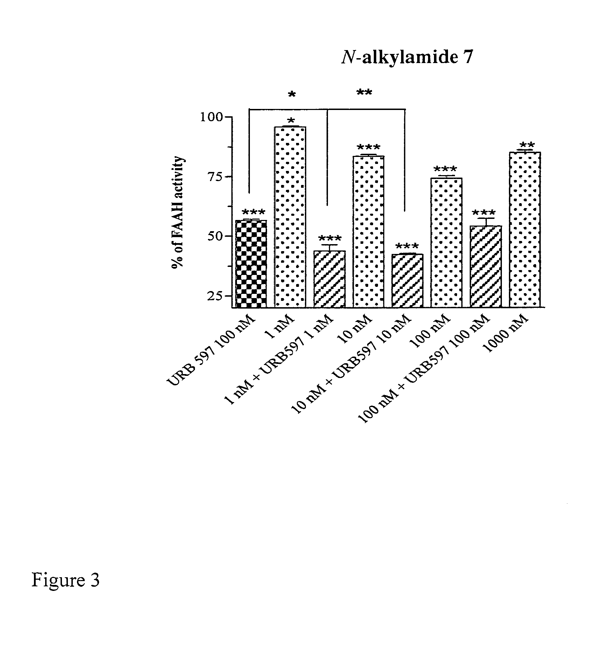

Figure 3: Effects of low nM concentrations of *N*-alkylamide **7** on FAAH activity alone and in combination with the FAAH inhibitor URB597. Example **7** showed synergistic effects with URB597. Data are mean values ± SEM of 3 independent experiments.

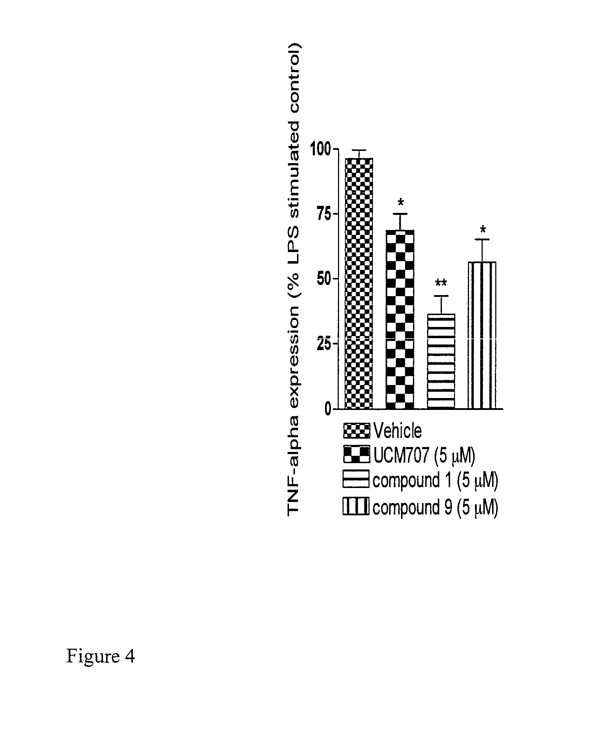

Figure 4: **1** and **9** inhibit LPS-induced TNF-alpha release into culture medium (measured by ELISA). Human macrophages (10⁶ cells) were stimulated with LPS (500 ng/ml) and treated with UCM707 and/or compounds **1** and **9** at 5 µM for 18 h. N = 3 ± SEM.

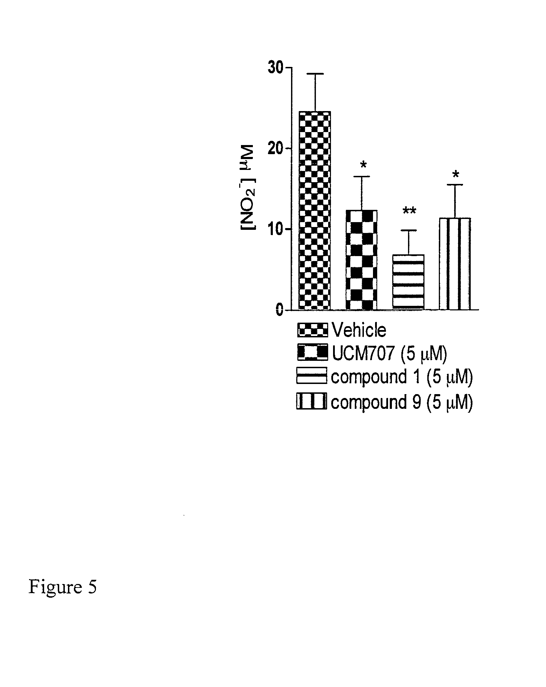

Figure 5: UCM707 (positive control) and compounds **1** and **9** diminish NO₂⁻ production. Human macrophages (10⁶ cells) were stimulated with LPS (500 ng/ml) and treated with UCM707 and/or compounds **1** and **9** at 5 µM for 18 h. Culture supernatants were assayed for nitrite levels, using the Griess reagent as a reflection of NO production. Results are the mean ± SEM of four independent experiments performed in duplicate

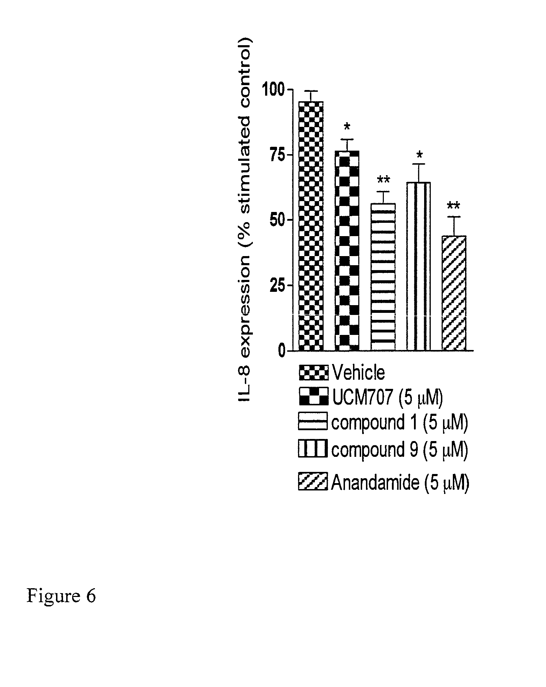

Figure 6: In CB receptor expressing human HaCaT keratinocytes (10⁶ cells) UCM707 (control) **1** and **9** like the endocannabinoid anandamide (each 5 µM) inhibited tolerogen (10 µg/ml) induced chemokine IL-8 (6h stimulation and incubation). N = 3 ± SEM.

### Detailed Description of the Invention

In accordance with the present invention the applicant has developed the new class of compounds of dodeca-*2E,4E*-diene amides and has surprisingly found out that these compounds can effectively be used as a medicament, preferably as a dermatologic agent, and as a cosmetic due to their lipophilic nature. The conditions and diseases to be treated are particularly inflammation, itching, pruritus, irritation, pain, oedema, and/or pro-allergic or allergic conditions. The compounds turned out to be effective not only in the treatment but also in the prevention of the above mentioned conditions. The conditions also comprise inflammatory reactions and irritation of skin and mucosa of a mammal, preferably of a human, e.g. caused by environmental stress and influences, such as UV irradiation, toxic substances, and allergens in general. Thus, the compounds have shown to effectively reduce several kinds of skin and mucosa discomforts as mentioned above. In particular, they have shown to provide for analgesic and anti-pruritic properties.

Without intending to be bound by any theory the applicant believes that the reason for the activity of dodeca-*2E,4E*-diene amides is based on their function as modulators of the endocannabinoid system. In this respect it is known that the endocannabinoids *N-*arachidonoylethanolamide (Anandamide, AEA) and 2-arachidonoylglycerol (2-AG) bind to G-protein-coupled CB₁ and CB₂ cannabinoid receptors. The CB₁ receptor is primarily (but not exclusively) expressed in neurons, the CB₂ receptor is mainly present in immunocytes, such as monocytes (splenocytes), macrophages, B cells, but also in astroglial cells. In the skin, CB receptors, in particular the CB₂ receptor, are expressed in keratinocytes and fibroblasts. Upon CB receptor activation, endocannabinoids are actively transported through the cell membrane and degraded. The metabolic enzymes fatty acid amide hydrolase (FAAH) and monoacyl glycerol lipase (MAGL) are responsible for the hydrolysis of AEA and 2-AG, respectively, thus leading to inactivation of these signaling molecules. While activation of the CB₁ receptor has been shown to mediate distinct neurophysiological effects, modulation of the CB₂ receptor by both agonists and inverse agonists is known to interfere with different inflammatory processes. CB₂ receptor activation can lead to anti-inflammatory effects *in vivo* and CB₂ receptor modulation has been implicated in the pathophysiology of a number of diseases, including chronic pain, inflammation of the gastrointestinal tract, osteoporosis, and liver diseases. Plants employ structurally similar lipid signaling molecules as animals to process cellular information, including endocannabinoid-like fatty acid derivatives. Since plants do not generally synthesize arachidonic acid, different *N*-acylethanolamines like e.g. *N-*palmitoylethanolamide are produced in plants and these lipids have also been shown to act on proteins directly or indirectly associated with the endocannabinoid system (ECS) in animals and humans, such as FAAH, the transient receptor potential vanilloid receptor (TRPV1) or the newly postulated cannabinoid receptor GPR55.

Thus, in accordance with the present invention it has been found that the dodeca-*2E,4E*-diene amides show a significant functional interference with the ECS which is assumed to be a reason for their pharmacological activity. In particular, the claimed dodeca-*2E,4E*-diene amides inhibit AEA re-uptake. In addition, some compounds of the invention also inhibit FAAH. In other words, these compounds inhibit AEA re-uptake in addition to or without inhibiting FAAH activity and without effecting CB₁ and CB₂ receptors.

The compounds of the present invention are represented by the following formula (1): 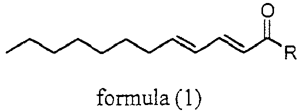 wherein R is a residue -NR¹R² with R¹ and R² being defined as in claim 1.

Suitable substituents of the carbon chains and rings in accordance with the invention are halogens, preferably F, Cl and Br and I, C₁-C₁₀ alkyl, C₁-C₁₀ alkoxy, C₆-C₂₀ aryl, hydroxyl, -SH, -SO₃H, amine groups, -COOH, and COOR', wherein R' is a C₁-C₁₀ alkyl or an alkali metal. In general, particularly preferred alkyl and alkoxy groups used as substituents in accordance with the present invention as described herein are C₁-C₆ alkyl and C₁-C₆ alkoxy groups, more preferably methyl, ethyl, n-propyl, i-propyl, n-butyl, i-butyl, n-pentyl, isopentyl, methoxy, ethoxy, n-propoxy, i-propoxy, n-butoxy, i-butoxy, n-pentoxy, iso-pentoxy. Similarly, particularly preferred aryl groups are C₆-C₁₀ aryl groups, most preferably phenyl. Suitable substituents are also selected from alkenyl, alkinyl, cycloalkyl, heteroaryl, heterocyclyl as defined herein below.

Suitable heteroatoms in accordance with the present invention are O, S, N, P, more preferred O and N.

In preferred compounds of the invention R¹ is hydrogen and R² is selected from the group of substituted or unsubstituted C₆-C₂₀ aryl, substituted or unsubstituted C₅-C₂₀ heteroaryl, substituted or unsubstituted C₆-C₂₀ arylalkyl or heteroarylalkyl, or hydroxy-C₁-C₂₀-alkyl.

In more preferred compounds of the present invention R¹ is hydrogen and R² is selected from hydroxy-C₁-C₂₀-alkyl, furanyl-C₁-C₁₀-alkyl, pyrrolyl-C₁-C₁₀-alkyl, (C₁-C₁₀-alkylpyrrolyl)-C₁-C₁₀-alkyl, benzyl, C₁-C₁₀-alkoxybenzyl, C₁-C₁₀-alkoxyphenalkyl, and biphenylyl.

In even more preferred compounds of the present invention R¹ is hydrogen and R² is selected from hydroxy-C₁-C₂₀-alkyl, furanylmethyl, (methylpyrrolyl)methyl, methoxybenzyl, methoxyphenethyl, and biphenylyl. Preferably, R² is selected from hydroxy-C₁-C₆-alkyl, furanylmethyl, (methylpyrrolyl)methyl, methoxybenzyl, methoxyphenethyl, and biphenylyl.

In particularly preferred compounds of the present invention R¹ is hydrogen and R² is selected from 2-hydroxyethyl, furan-3-ylmethyl, (1-methyl-1*H*-pyrrol-3-yl)methyl, 2-methoxybenzyl, 3-methoxybenzyl, 4-methoxybenzyl, 3-methoxyphenethyl, and biphenyl-4-yl.

According to the present invention, in compounds of formula (1), R may also be selected from the group consisting of the following radicals (which may be substituted by substituent groups as defined herein):

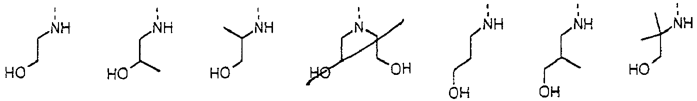

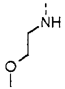

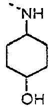

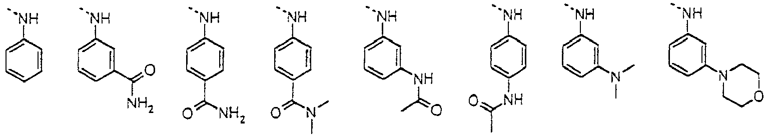

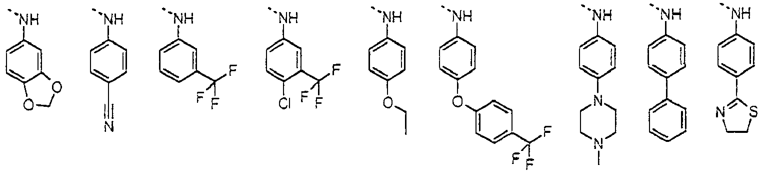

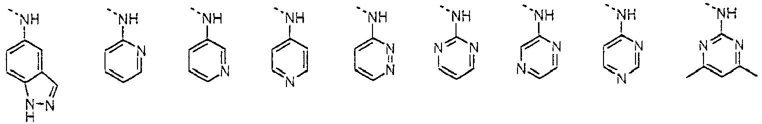

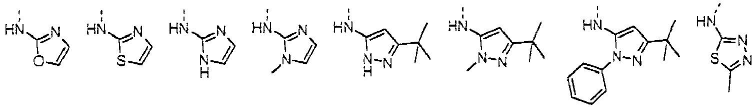

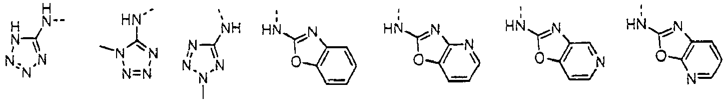

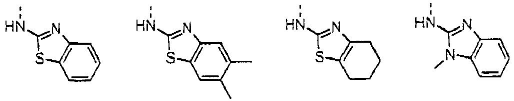

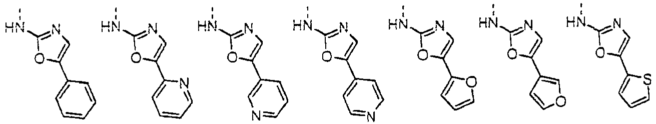

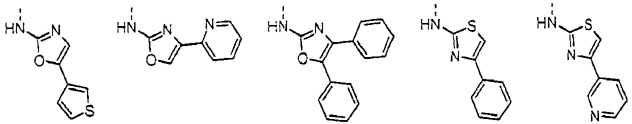

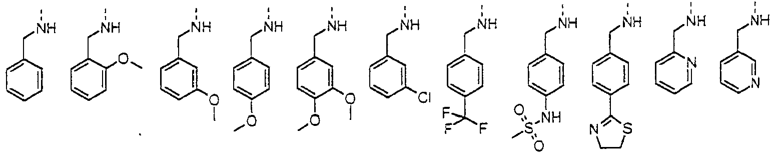

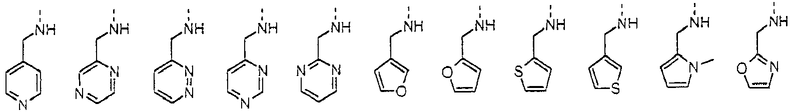

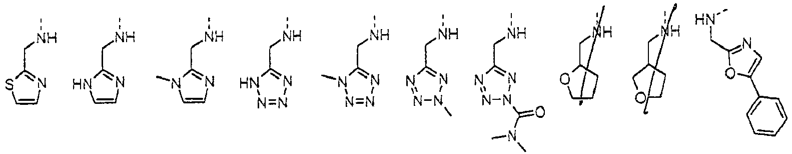

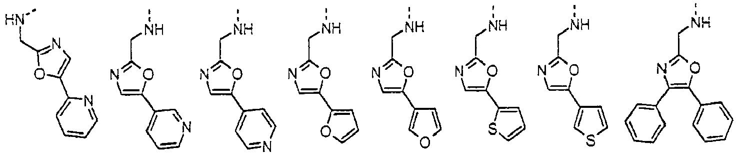

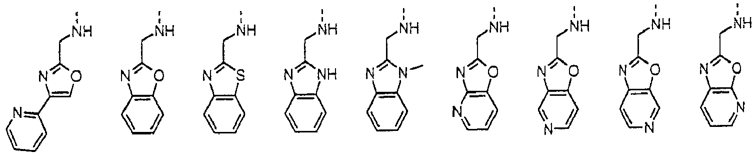

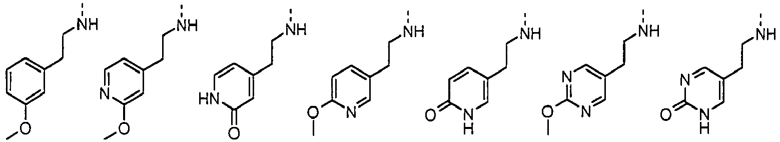

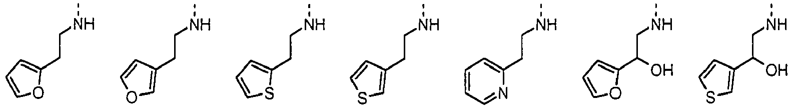

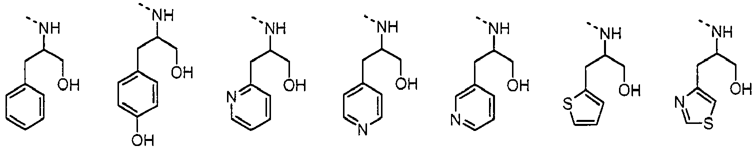

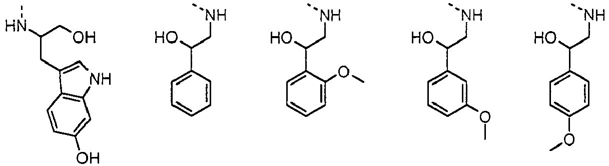

In particular, in accordance with the present invention, the employed terms for defining the groups R, R¹ and R² as well as the respective substituents of the carbon chains and rings have the meaning as described below:
Alkyl is a straight chain or branched alkyl having 1, 2, 3, 4, 5 or 6 carbon atoms, such as methyl, ethyl, n-propyl, isopropyl, n-butyl, isobutyl, tert-butyl, n-pentyl, isopentyl, neopentyl, or hexyl.
Alkenyl is a straight chain or branched alkyl having 2, 3, 4, 5 or 6 carbon atoms and one to three double bonds, preferably one or two double bonds, most preferably one double bond. Preferred examples of a C₂₋₆ alkenyl group are ethenyl, prop-1-enyl, pro-2-enyl, isoprop-1-enyl, n-but-1-enyl, n-but-2-enyl, n-but-3-enyl, isobut-1-enyl, isobut-2-enyl, n-pent-1-enyl, n-pent-2-enyl, n-pent-3-enyl, n-pent-4-enyl, n-pent-1,3-enyl, isopent-1-enyl, isopent-2-enyl, neopent-1-enyl, n-hex-1-enyl, n-hex-2-enyl, n-hex-3-enyl, n-hex-4-enyl, n-hex-5-enyl, n-hex-1,3-enyl, n-hex-2,4-enyl, n-hex-3,5-enyl, and n-hex-1,3,5-enyl. More preferred examples of a C₂₋₆ alkenyl group are ethenyl and prop-1-enyl.
Alkinyl is a straight chain or branched alkyl having 2, 3, 4, 5 or 6 carbon atoms and one to three triple bonds, preferably one or two triple bonds, most preferably one triple bond. Preferred examples of a C₂₋₆ alkinyl group are ethinyl, prop-1-inyl, pro-2-inyl, n-but-1-inyl, n-but-2-inyl, n-but-3-inyl, n-pent-1-inyl, n-pent-2-inyl, n-pent-3-inyl, n-pent-4-inyl, n-pent-1,3-inyl, isopent-1-inyl, neopent-1-inyl, n-hex-1-inyl, n-hex-2-inyl, n-hex-3-inyl, n-hex-4-inyl, n-hex-5-inyl, n-hex-1,3-inyl, n-hex-2,4-inyl, n-hex-3,5-inyl, and n-hex-1,3,5-inyl. More preferred examples of a C₂₋₆ alkinyl group are ethinyl and prop-1-inyl.
Cycloalkyl is an alkyl ring having 3, 4, 5, 6 or 7 carbon atoms at the most, such as cyclopropyl, cyclobutyl, cyclopentyl, cyclohexyl or cycloheptyl, more preferably 3, 4, 5 or 6 carbon atoms.
Heteroaryl is an aromatic moiety having 1, 2, 3, 4 or 5 carbon atoms and at least one heteroatom selected from O, N and/or S and is preferably selected from thienyl, pyrrolyl, imidazolyl, pyrazolyl, pyridyl, pyrazinyl, pyrimidinyl, pyridazinyl, isothiazolyl, isoxazyl, furanyl, and indazolyl, more preferably from thienyl, furanyl, imidazolyl, pyridyl, and pyrimidinyl.
Heterocyclyl is a saturated or unsaturated ring containing at least one heteroatom selected from O, N and/or S and 1, 2, 3, 4, 5, 6 or 7 carbon atoms. Preferably, heterocyclyl comprises at most 11 ring atoms (including carbon and heteroatoms) and more preferably is a 4 to 8-membered ring and even more preferably is selected from tetrahydrofuranyl, azetidinyl, pyrrolidinyl, piperidinyl, pyranyl, morpholinyl, and thiomorpholinyl, more preferably from piperidinyl and pyrrolidinyl.
Halogen is a halogen atom selected from F, Cl, Br and I, preferably from F, Cl and Br.

Preferred compounds according to the invention are also those wherein R¹ is hydrogen and R² is selected from the group of hydroxyalkyl (e.g. hydroxyethyl and hydroxypropyl), arylalkyl (e.g. benzyl, methoxyphenethyl, methoxyphenylmethyl), aryl (e.g. phenyl), heteroarylalkyl (e.g. furanylethyl, thiophenylethyl, thiophenylmethyl, tetrazolylmethyl and pyridylethyl) and heteroaryl (e.g. thiazolyl, benzothiazolyl, pyridazinyl, and pyrimidinyl). These groups may be further substituted by substituents as described herein.

The compounds according to the present invention are not those wherein -NR¹R² is -NHCH₂CH(CH₃)₂, -NHCH₂CH(CH₃)CH₂CH₃, -NH(CH₂)₂CH(CH₃)₂, unsubstituted pyrrolidinyl, and unsubstituted piperidinyl. In other words, dodeca-*2E,4E*-diene isobutyl amide, dodeca-*2E,4E*-diene 2-methylbutyl amide, dodeca-*2E,4E*-diene 3-methylbutyl amide, dodeca-*2E,4E*-diene pyrrolidyl amide (i.e. unsubstituted at the pyrrolidin ring), and dodeca-*2E*,*4E*-diene piperidinyl amide (i.e. unsubstituted at the piperidin ring) are excluded from the present invention. Additionally, dodeca-*2E,4E*-diene p-hydroxyphenethyl amide is preferably also excluded from the present invention.

Furthermore, also compounds are excluded from the present invention wherein R¹ is hydrogen and R² comprises a -CH-C(=O)- moiety directly bound via the -CH group to the N atom of -NR¹R². In other words, all compounds comprising an α-amino acid group directly connected via the N atom with the carbonyl C atom at the dodeca-*2E,4E*-diene moiety are excluded from the present invention. Furthermore, the compounds according to the present invention do not contain a 12-membered (mono- or poly-) unsaturated heterocyclic ring.

Some of these compounds are already disclosed in the prior art as active ingredients in pharmaceutical compositions, however, they are not known to be suitable in the treatment/prevention of the skin disorders as described herein.

The compounds of structural formula (1) are effective as modulators of the endocannabinoid system and are particularly effective as inhibitors of AEAT (AEA transport). They are useful for the treatment and/or prevention of disorders responsive to inhibition of AEAT, such as inflammation, irritation, itching, pruritus, pain, oedema and/or pro-allergic, allergic conditions and other diseases in particular with AEAT involvement.

### Optical Isomers - Diastereomers - Geometric Isomers - Tautomers

Compounds of structural formula (1) contain one or more asymmetric centers and can occur as racemates and racemic mixtures, single enantiomers, diastereomeric mixtures and individual diastereomers. The present invention is meant to comprehend all such isomeric forms of the compounds of structural formula (1).

Compounds of structural formula (1) may be separated into their individual diastereomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (1) may be obtained by stereospecific synthesis using optically pure starting materials or reagents of known absolute configuration.

### Salts

The compounds according to the invention can be present in the form of pharmaceutical acceptable salts. The term "pharmaceutical acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzene sulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

### Administration, Dose Ranges and Formulation

The compounds of the invention are incorporated into pharmaceutical or cosmetic preparations by admixing them with a pharmaceutically or cosmetically acceptable carrier or excipient. Suitable carriers are known to the skilled person. Preferably the composition is a dermatological composition suitable to be applied topically on the skin or mucosa of a mammal. The form of the composition is not particularly limited, however, preferred forms are emulsions, suspensions and solutions. In certain embodiments, the compositions are in the form of lotions, creams, gels, solutions, sprays, cleansers, powders, ointments, waxes, lipsticks, soaps, shampoos, hydroalcoholic solutions, suspensions, foams, scrubs, saturated pads, skin or hair conditioning agents.

The compositions according to the invention, however, can be applied in any other way known to the skilled person such as oral or parenteral. Non-limiting examples are sublingual, vaginal, rectal, intravenous, nasal, intramuscular, inhalative, ocular and percutaneous. Suitable carriers and excipients are commonly known to the skilled person.

Compositions in accordance with the present invention may contain the diene amide compounds in amounts of 0.001 to 40% by weight of the composition, preferably 0.01 to 5 % by weight, more preferably 0.1 to 2 % by weight and most preferably 0.5 to 1.5 % by weight.

Compositions in accordance with the present invention may contain cosmetically and pharmaceutically/dermatologically acceptable auxiliaries usually employed in cosmetic and pharmaceutical preparations and known to the skilled person. These include for example preservatives, bactericides, perfumes, thickeners, emulsifiers, surfactants, softening agents, moisturizing agents, oils, fats, waxes, organic solvents, water, alcohols, polyols, polymers, foam stabilizers, anti-foaming agents, penetration enhancers or other conventional components of a pharmaceutical or cosmetic preparation.

### Preparation of Compounds of the Invention

The compounds of formula (1), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (1) may be obtained by stereospecific syntheses using optically pure starting materials or reagents of known configuration.

The compounds of formula (1) of the present invention can be prepared according to the procedures of the following schemes and examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. Compounds containing a basic or acidic group can be further converted into the form of their pharmaceutical acceptable salts, such as described previously. All temperatures are degrees Celsius.

In the schemes, preparations and examples below, various reagent symbols and abbreviations have the following meanings:

| | |
|---|---|
| AcOH | acetic acid |
| Boc | tert-butoxycarbonyl |
| DCM | dichloromethane |
| DIEA | ethyl-diisopropylamine |
| DMAP | 4-dimethylaminopyridine |
| DMF | N,N-dimethylformamide |
| Et₂O | diethyl ether |
| EtOH | ethanol |
| h | hour(s) |
| HATU | O-(7-azabenzotriazol-1-yl)-N,N,N',N'-tetramethyluronium hexafluorophosphate |
| HRMS | high-resolution mass spectrometry |
| m/z | mass-to-charge ratio |
| MeOH | methanol |
| mp | melting point |
| MW | molecular weight |
| NMR | nuclear magnetic resonance |
| RT | room temperature |
| TEA | triethylamine |
| THF | tetrahydrofurane |
| TLC | thin layer chromatography |
| t_{R} (min) | HPLC retention time |

### Reaction Scheme 1:

### Synthesis of (2E,4E)-dodeca-2,4-dienoic acid using a Wittig-Horner reaction

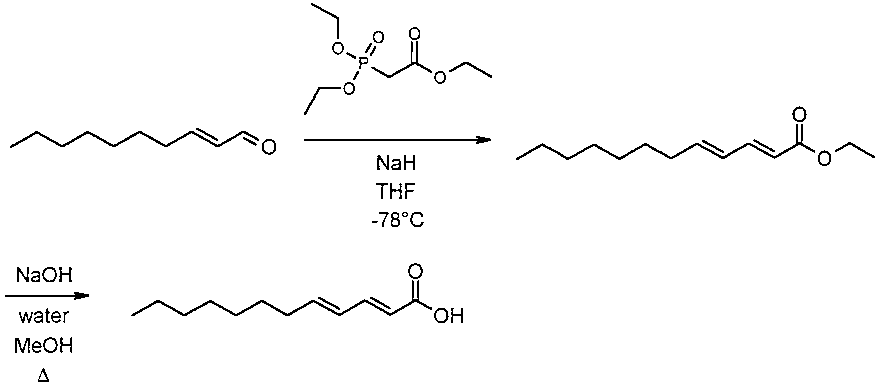

The starting material for the synthesis of compounds of the present invention, (*2E*,*4E*)-dodeca-2,4-dienoic acid, can be obtained by reacting (*2E*)-dec-2-enal with a trialkylphosphonoacetate in the presence of a base such as sodium hydride or butyl lithium in an inert solvent like tetrahydrofurane at an appropriate temperature. The ester thus obtained can be hydrolyzed in the presence of a base such as sodium hydroxide or lithium hydroxide in suitable solvent such as a mixture of water and methanol or water and THF at a given temperature.

### Reaction Scheme 2:

### Oxidation of (2E,4E)-dodeca-2,4-dienal

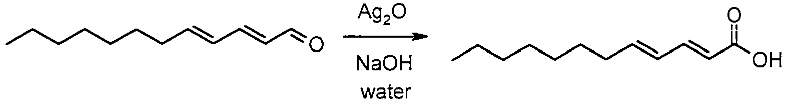

Alternatively, (*2E*,*4E*)-dodeca-2,4-dienal can be reacted with an oxidizing agent such as silver oxide in the presence of a base like sodium hydroxide in a suitable solvent such as water or a mixture of THF and water to yield (*2E,4E*)-dodeca-2,4-dienoic acid.

### Reaction Scheme 3:

### Acid chloride formation

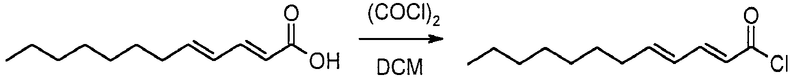

(*2E,4E*)-Dodeca-2,4-dienoic acid can be converted to the corresponding acid chloride by reaction with a reagent such as oxalyl chloride or thionyl chloride in a suitable solvent like DCM.

### Reaction Scheme 4:

### Amine synthesis involving a Henry reaction

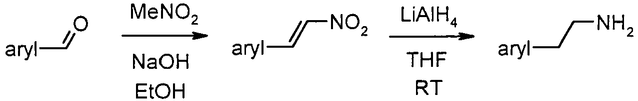

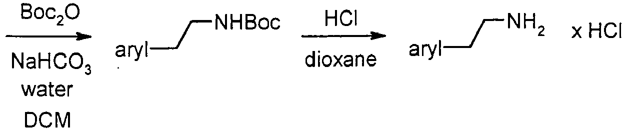

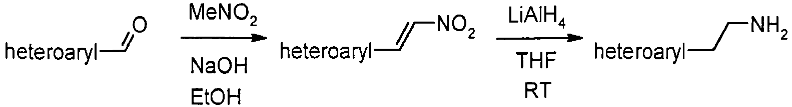

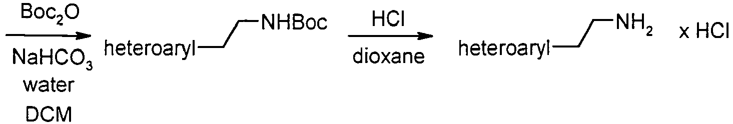

Optionally substituted 2-arylethylamines can be obtained as depicted in Reaction Scheme 4. Reaction of an optionally substituted arylcarboxaldehyde with nitromethane in a solvent like ethanol in the presence of sodium hydroxide or ammonium acetate yields the corresponding nitroalkenes. Subsequent reduction with lithium aluminium hydride in dioxane at room temperature yields the targeted amines.

In some cases the amines are Boc-protected for purification. The protective group is introduced by reacting the amine with di-tert-butyl dicarbonate in the presence of a base such as sodium bicarbonate in a suitable solvent such as a mixture of water and DCM. Following the purification process the amine can be obtained after deprotection with an acid such as hydrogen chloride in dioxane. The amine can be isolated as its hydrochloride salt or the free base can be liberated.

The same reaction sequence can also be performed using an optionally substituted heteroarylcarboxaldehyde.

### Reaction Scheme 5:

### Synthesis of amino alcohols

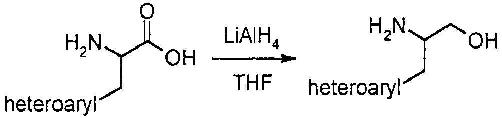

Reaction Scheme 5 shows the synthesis of optionally substituted heterorylalaninol derivatives. Optionally substituted heteroaryalanine is reacted with a reducing agent such as lithium aluminium hydride or borane in an inert solvent like tetrahydrofurane or diethyl ether at a suitable temperature to yield the corresponding amino alcohol.

### Reaction Scheme 6:

### Alternative routes for the synthesis of amino alcohols

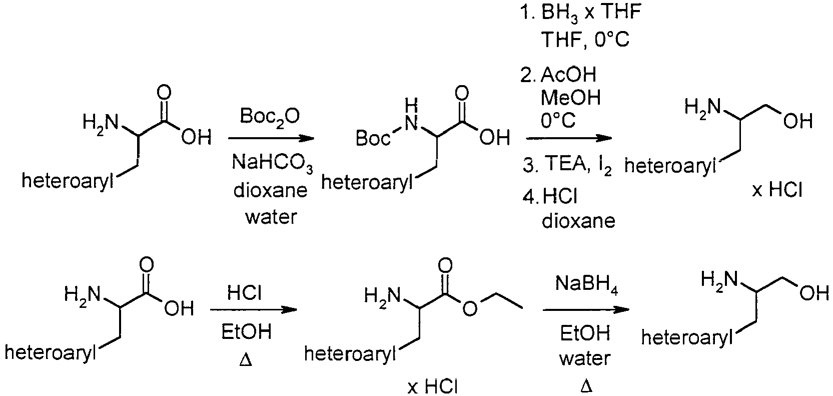

In some cases the direct reduction of an optionally substituted heteroarylalanine is not possible. In such cases a derivative of the amino acid is prepared and this intermediate is subjected to a reduction reaction, as depicted in Reaction Scheme 6.

For example a Boc-protecting group can be introduced using a reagent like di-tert-butyldicarbonate in an appropriate solvent such as a mixture of dioxane and water in the presence of a base like sodium bicarbonate. Reduction of the acid function can be accomplished by treatment with a reagent such as borane tetrahydrofurane complex in a solvent like THF at a suitable temperature. The reaction mixture can be hydrolyzed by using a mixture of acetic acid and methanol at low temperature. The crude product can subsequently been treated with iodine in the presence of a base such as triethylamine. Following the reduction, deprotection of the amine function can be accomplished by reaction of the intermediate product with an acid like hydrochloric acid in a solvent such as dioxane. The free base may be liberated subsequently or the hydrochloride can be used for the following reactions.

Alternatively, optionally substituted heteroarylalanine can be converted to the corresponding amino ester hydrochloride. Esterification can be achieved by heating the amino acid in a suitable alcohol like methanol or ethanol in the presence of hydrochloric acid, paratoluenesulfonic acid or thionyl chloride. The ester function can be reduced using a reagent such as sodium borohydride in a solvent like a mixture of ethanol and water at elevated temperature. Ester function can also been performed using a reagent such as borane or litium aluminium hydrid in an inert solvent such as tetrahydrofurane or diethyl ether.

### Reaction Scheme 7:

### Synthesis 2-heteroarylethylamines using a Curtius rearrangement

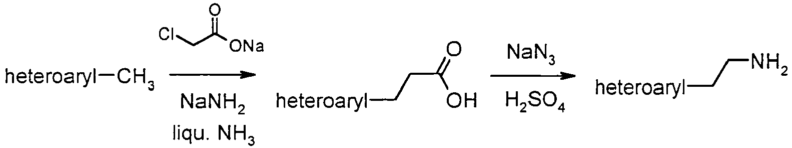

As shown in Reaction Scheme 7, optionally substituted methyl-substituted heteroaromatic compounds can be reacted with a reagent such as sodium chloroacetate in the presence of sodium amide in liquid ammonia to yield the corresponding heteroarylpropionic acids. Subsequent reaction with sodium azide in the presence sulfuric acid provides the target amines.

### Reaction Scheme 8:

### Coupling of (2E,4E)-dodeca-2,4-dienoic acid with amines

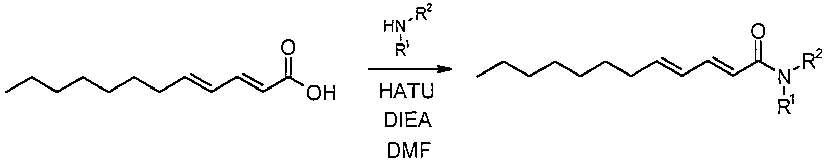

(*2E,4E*)-Dodeca-2,4-dienoic acid, obtained as described in Reaction Schemes 1 and 2, respectively, can be coupled with amines HNR¹R² using an appropriate coupling reagent such as HATU in a suitable solvent like DMF in the presence or without a base like DIEA. Alternatively, the acid can also been activated with other suitable reagents, for example by formation of a mixed anhydride using a reagent like ethyl chloroformate.

### Reaction Scheme 9:

### Reaction of (2E,4E)-dodeca-2,4-dienoyl chloride with amines

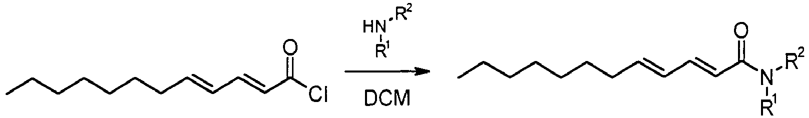

An alternative pathway leading to the target molecules starts with (2E,*4E*)-dodeca-2,4-dienoyl chloride. Reaction with an amine HNR¹R² in an inert solvent like DCM yields the desired products. The reaction can be carried out in the presence or without a base such as DMAP.

### Analyses

Compounds of the present invention were analyzed using various analytical methods. These methods are summarized below.

### Melting points

Melting points (mp) were determined in open capillaries using a Büchi Melting Point B-540 apparatus.

### NMR spectra

Nuclear magnetic resonance (¹H- and ¹³C-NMR) spectra were recorded on a Bruker Avance 400 MHz spectrometer and were analyzed using the Topspin 1.3 software. Chemical shifts (δ) are reported in parts per million (ppm) relative to CDCl₃, which was set to 7.26 ppm for the analysis of the ¹H-spectra and to 77.00 ppm for the evaluation of the ¹³C-spectra, the coupling constants (*J*) were expressed in hertz. Signals were quoted as the following: s, singlet; d, doublet; t, triplet; m, muliplet.

### GC- and HPLC-analysis coupled with high-resolution MS

High-resolution ESI positive mass spectra were obtained on a 4.7T Ultima FT-ICR-ESI Mass spectrometer (Varian, Inc., Palo Alto, CA, USA) with ionization voltage of 3.0-3.8 kV. The purity of the *N*-alkyl amides (Example 1, Examples 3-10 and Compounds A-E) was assessed by gas chromatography using a 15 m x 0.25 mm, 0.25 µm film, ZB-5 column. Two temperature profiles have been applied to detect impurity. System A: The oven was held at 55°C for 3 min, and the temperature was then increased at a rate of 6°C/min to 230°C and held for 10 min. System B: The temperature was increased from 150°C at a rate of 6°C/min to 230°C and held for 20 min. Since Example 2 decomposed during gas chromatography, the purity of Example 2 was evaluated using high performance liquid chromatography (HPLC) on a Waters Symmetry^{®} C18 column (4.6 x 100 mm) with detection by a UV diode array detector. Two different solvent-systems were used to display purity. System A: The mobile phase was water and acetonitrile using the following gradient elution program: initial 60% acetonitrile for 2 min, then a linear gradient to 95% acetonitrile over 7 min, which was held for 1 min, followed by a linear gradient to 60% acetonitrile over 1 min, which was held for 1 min. System B: The same gradient elution program was adopted as in system A but using methanol instead of acetonitrile. The purity is displayed as the average of the two applied systems.

### Analytical LC-MS

### Analytical conditions summary:

Agilent 1100 with ELSD (PL-ELS 2100) and diode array detector with UV-detection between 220 and 320 nm and Mass Selective Detector (MSD) in ESI+ and ESI- modus (mass range:
m/z = 100-800),
Column: Waters Xbridge C18, 3.5 *µ*m, 2.1 mm x 50 mm,
linear gradient with acetonitrile in water (0.1% HCOOH)
Flow rate of 0.8 ml/min; column temperature: 30°C;

| | |
|---|---|
| Mobile Phase A: | acetonitrile (0.1% HCOOH) |
| Mobile Phase B: | water (0.1% HCOOH) |

or
Agilent 1200 with diode array detector with UV-detection between 220 and 320 nm and Mass Selective Detector (MSD) in ESI+ and ESI- modus (mass range: m/z = 100-800),
Column: Waters Xbridge C18, 3.5 *µ*m, 2.1 mm x 50 mm, linear gradient with acetonitrile in water (0.1% HCOOH)
Flow rate of 0.8 ml/min; column temperature: 30°C;

| | |
|---|---|
| Mobile Phase A: | acetonitrile (0.1% HCOOH) |
| Mobile Phase B: | water (0.1% HCOOH) |

### Gradient A:

linear gradient from 2% to 98% acetonitrile in water (0.1% HCOOH)

| | |
|---|---|
| 0.0 min | 2% A |
| 3.5 min | 98% A |
| 6.0 min | 98% A |

The following describes the detailed examples of the invention which can be prepared via reaction schemes I to 9.

**Table 1:**

| | | | | | |
|---|---|---|---|---|---|
| 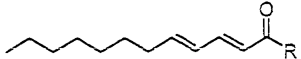 | | | | | |

| | | HPLC¹⁾ | | MS¹⁾ | |
|---|---|---|---|---|---|
| No. | R | t_{R} (min) | method | MW (calc.) free base | [M+H⁺] (found) |
| 1 | 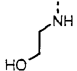 | n.a. | n.a. | n.a. | n.a. |
| 4 | 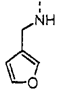 | n.a. | n.a. | n.a. | n.a. |
| 5 | 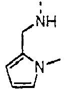 | n.a. | n.a. | n.a. | n.a. |
| 6 | 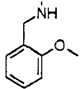 | n.a. | n.a. | n.a. | n.a. |
| 7 | 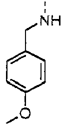 | n.a. | n.a. | n.a. | n.a. |
| 8 | 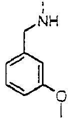 | n.a. | n.a. | n.a. | n.a. |
| 9 | 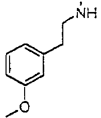 | n.a. | n.a. | n.a. | n.a. |
| 10 | 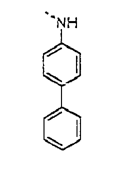 | n.a. | n.a. | n.a. | n.a. |
| 11 | 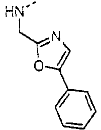 | 4.08 | A | 352.48 | 353 |
| 12 | 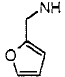 | 3.96 | A | 275.39 | 276 |
| 14 | 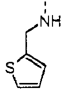 | 4.05 | A | 291.46 | 292 |
| 15 | 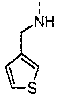 | 4.04 | A | 291.46 | 292 |
| 16 | 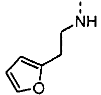 | 4.02 | A | 289.42 | 290 |
| 17 | 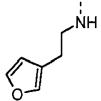 | 4.01 | A | 289.42 | 290 |
| 18 | 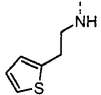 | 4.10 | A | 305.49 | 306 |
| 19 | 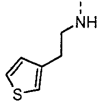 | 4.09 | A | 305.49 | 306 |
| 20 | 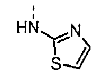 | 4.02 | A | 278.42 | 279 |
| 21 | 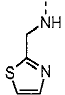 | 3.78 | A | 292.45 | 293 |
| 22 | 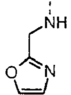 | 3.71 | A | 276.38 | 277 |
| 23 | 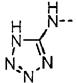 | 3.71 | A | 263.35 | 264 |
| 24 | 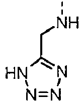 | 3.56 | A | 277.37 | 278 |
| 25 | 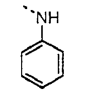 | 4.17 | A | 271.41 | 272 |
| 26 | 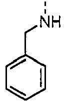 | 4.09 | A | 285.43 | 286 |
| 27 | 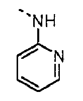 | 3.84 | A | 272.39 | 273 |
| 28 | 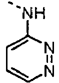 | 3.84 | A | 273.38 | 274 |
| 29 | 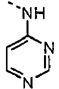 | 3.89 | A | 273.38 | 274 |
| 30 | 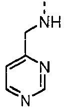 | 3.61 | A | 287.41 | 288 |
| 31 | 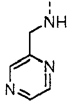 | 3.67 | A | 287.41 | 288 |
| 32 | 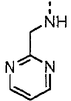 | 3.64 | A | 287.41 | 288 |
| 33 | 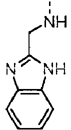 | 3.22 | A | 325.46 | 326 |
| 34 | 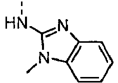 | 3.50 | A | 325.46 | 326 |
| 35 | 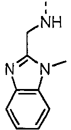 | 3.26 | A | 339.48 | 340 |
| 36 | 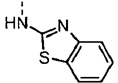 | 4.34 | A | 328.48 | 329 |
| 37 | 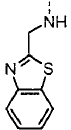 | 4.21 | A | 342.51 | 343 |
| 38 | 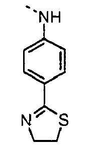 | 3.94 | A | 356.53 | 357 |
| 39 | 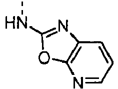 | 3.84 | A | 313.40 | 314 |
| 40 | 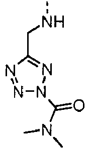 | 3.79 | A | 348.45 | 349 |
| 41 | 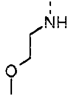 | 3.75 | A | 253.39 | 254 |
| 42 | 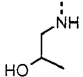 | 3.58 | A | 253.39 | 254 |
| 43 | 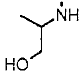 | 3.60 | A | 253.39 | 254 |
| 44 | 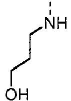 | 3.56 | A | 253.39 | 254 |
| 45 | 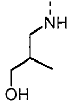 | 3.70 | A | 267.42 | 268 |
| 46 | 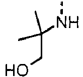 | 3.83 | A | 267.42 | 268 |
| 47 | 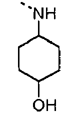 | 3.67 | A | 293.45 | 294 |
| 48 | 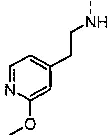 | 3.87 | A | 330.47 | 331 |
| 49 | 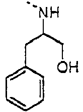 | 3.90 | A | 329.49 | 330 |
| 50 | 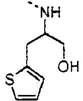 | 3.88 | A | 335.51 | 336 |
| 51 | 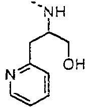 | 3.09 | A | 330.47 | 331 |
| 52 | 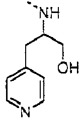 | 3.01 | A | 330.47 | 331 |
| 53 | 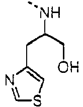 | 3.60 | A | 336.50 | 337 |
| 54 | 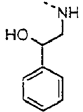 | 3.90 | A | 315.46 | 316 |
| 55 | 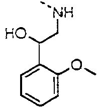 | 3.94 | A | 345.49 | 346 |
| 56 | 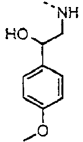 | 3.87 | A | 345.49 | 346 |
| 66 | 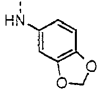 | 4.13 | A | 315.42 | 316 |
| 67 | 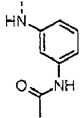 | 3.87 | A | 328.46 | 329 |
| 68 | 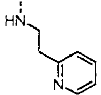 | 3.18 | A | 300.45 | 301 |
| 69 | 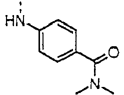 | 3.89 | A | 342.49 | 343 |
| 70 | 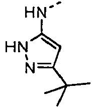 | 4.03 | A | 317.48 | 318 |
| 71 | 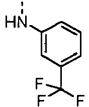 | 4.43 | A | 339.40 | 340 |
| 72 | 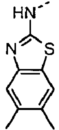 | 4.55 | A | 356.53 | 357 |
| 73 | 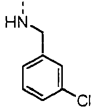 | 4.24 | A | 319.88 | 320 |
| 74 | 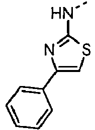 | 4.46 | A | 354.52 | 355 |
| 75 | 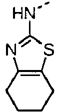 | 4.36 | A | 332.51 | 333 |
| 76 | 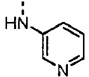 | 3.42 | A | 272.39 | 273 |
| 77 | 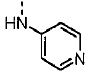 | 3.20 | A | 272.39 | 273 |
| 78 | 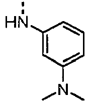 | 3.90 | A | 314.47 | 315 |
| 79 | 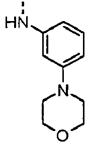 | 4.10 | A | 356.51 | 357 |
| 80 | 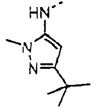 | 4.14 | A | 331.51 | 332 |
| 81 | 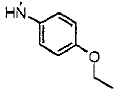 | 4.23 | A | 315.46 | 316 |
| 83 | 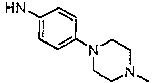 | 3.30 | A | 369.55 | 370 |
| 84 | 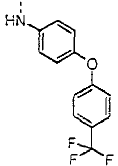 | 4.62 | A | 431.50 | 432 |
| 85 | 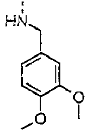 | 3.96 | A | 345.49 | 346 |

| | | | | | |
|---|---|---|---|---|---|
| ¹⁾ For examples 1 to 10 analytical data is provided in the detailed description of the preparation (see below). | | | | | |

The following examples are provided to illustrate the invention and are not limiting the scope of the invention in any manner.

### General procedure for the synthesis of examples 1 to 10 and compounds A to E.

Sodium hydride 60% (39 mmol) was solved in THF at 0°C. After addition of triethyl phosphonoacetate (39 mmol) the reaction mixture was stirred for 30 min at room temperature. Then (*E*)-dec-2-enal (32.4 mmol) was added dropwise at -78°C and let react for another 30 min. The ester was extracted with ethyl acetate and purified by column chromatography on silica gel. After saponifying the ester with 2M NaOH at 60°C for 3h in MeOH, the reaction mixture was acidified (pH = 2) by adding diluted HCl. The acid was extracted with ethyl acetate and the solvent was evaporated. Without further purification the acid was converted into the acid chloride by adding thionyl chloride and reflux in Et₂O for 90 min. After removing the excess of thionyl chloride in high vacuum, the appropriate amine (1.5 equivalent) was added slowly at room temperature to the acid chloride solved in Et₂O and let stirring for 60 min at room temperature. After no further conversion (checked by TLC) was observed (after 1-2 h), the reaction was stopped by adding saturated NaHCO₃ solution and the amide was extracted with ethyl acetate. The dried crude products were finally purified by flash column chromatography and analyzed by MS and NMR.

### Example 1: (2E,4E)-N-(2-hydroxyethyl)dodeca-2,4-diene amide (1).

White solid. Mp 89.3-92.3°C. HRMS (ESI) calcd for C₁₄H₂₅NO₂ (M + Na⁺) *m*/*z* 262.1778 found 262.1774. ¹H NMR (CDCl₃) δ (ppm): 7.22 (dd, 1H, *J =* 9.7, *J* = 14.9 Hz), 6.03-6.20 (m, 3H), 5.79 (d, 1H, *J* = 14.9 Hz), 3.75 (t, 2H, *J* = 5.2 Hz), 3.47-3.53 (m, 2H), 3.18 (s br, 1H), 2.14 (dd, 2H, *J* = 6.7 Hz, *J* = 13.7 Hz), 1.36-1.46 (m, 2H), 1.20-1.34 (m, 8H), 0.84-0.92 (m, 3H). ¹³C NMR (CDCl₃) δ (ppm): 14.07, 22.61, 28.75, 29.08, 29.13, 31.76, 32.98, 42.73, 62.56, 120.81, 128.08, 142.24, 144.09, 167.75.

### Example 4: (2E,4E)-N-(furan-3-ylmethyl)dodeca-2,4-diene amide (4).

White solid. Mp 108.9-110.2°C. HRMS (ESI) calcd for C₁₇H₂₅NO₂ (M + Na⁺) *m*/*z* 298.1778 found 298.1781. ¹H NMR (CDCl₃) δ (ppm): 7.37 (s, 2H), 7.21 (dd, 1H, *J* = 10.3 Hz, *J* = 15.1 Hz), 6.36 (s, br 1H), 5.76 (s, 1H), 6.02-6.14 (m, 2H), 5.73 (d, 1H, *J* = 16.9 Hz), 5.66 (s, 1H), 4.36 (d, 2H, *J=* 5.4 Hz), 2.14 (dd, 2H, *J =* 6.9 Hz, *J* = 12.9 Hz), 1.35-1.46 (m, 2H), 1.19-1.34 (m, 8H), 0.83-0.91 (m, 3H). ¹³C NMR (CDCl₃) δ (ppm): 14.06, 22.61, 28.76, 29.08, 29.12, 31.75, 32.96, 34.50, 110.32, 121.10, 122.25, 128.10, 140.16, 141.89, 143.45, 143.75, 166.23.

### Example 5: (2E,4E)-N-((1-methyl-1H-pyrrol-3-yl)methyl)dodeca-2,4-diene amide (5).

Brown solid. Mp 99.8-101.4°C. HRMS (ESI) calcd for C₁₈H₂₈N₂O (M + Na⁺) *m*/*z* 311.2094 found 311.2098. ¹H NMR (CDCl₃) δ (ppm): 7.21 (dd, 1H, *J* = 10.0 Hz, *J* = 15.2 Hz), 6.60 (t, 1H, *J* = 2.2 Hz), 6.01-6.13 (m, 4H), 5.70 (d, 1H, *J* = 15.8 Hz), 5.60 (s, br 1H), 4.49 (d, 2H, *J* = 5.5 Hz), 3.56 (s, 3H), 2.14 (dd, 2H, *J=* 6.6 Hz, *J=* 12.9 Hz), 1.35-1.47 (m, 2H), 1.20-1.34 (m, 8H), 0.83-0.92 (m, 3H). ¹³C NMR (CDCl₃) δ (ppm): 14.05, 22.59, 28.74, 29.06, 29.06, 31.73, 32.93, 33.76, 35.14, 106.95, 108.80, 121.10, 123.09, 128.09, 128.67, 141.87, 143.73, 165.70.

### Example 6: (2E,4E)-N-(2-methoxybenzyl)dodeca-2,4-diene amide (6)

White solid. 99.1 %. Mp 112.5-114.4°C. HRMS (ESI) calcd for C₂₀H₂₉NO₂ (M + Na⁺) *m*/*z* 338.2090 found 338.2092. ¹H NMR (CDCl₃) δ (ppm): 7.22-7.30 (m, 2H), 7.18 (dd, 1H, *J* = 9.9 Hz, *J=* 15.0 Hz), 6.90 (dt, 1H, *J=* 1.0 Hz, *J=* 7.5 Hz), 6.86 (d, 1H, *J=* 8.4 Hz), 6.00-6.19 (m, 2H), 5.9 (s br 1H), 5.73(d, 1H, *J=* 15.0 Hz), 4.51 (d, 2H, *J=* 5.8 Hz), 3.85 (s, 3H), 2.12 (dd, 2H, *J* = 6.8 Hz, *J =* 13.3 Hz), 1.18-1.33 (m, 10H), 0.83-0.91 (m, 3H). ¹³C NMR (CDCl₃) δ (ppm): 14.05, 22.60, 28.78, 29.07, 29.10, 31.74, 32.92, 39.36, 55.3, 110.25, 120.71, 121,73, 126.33, 128.21, 128.80, 129.89, 141.35, 143.14, 157.52, 166.05.

### Example 7: (2E,4E)-N-(4-methoxybenzyl)dodeca-2,4-diene amide (7)

White solid. Mp 125.1-126.2°C. HRMS (ESI) calcd for C₂₀H₂₉NO₂ (M + Na⁺) *m*/*z* 388.2090 found 388.2092. ¹H NMR (CDCl₃) δ (ppm): 7.23-7.26 (m, 1H), 7.17-7.23 (m, 2H), 6.60-6.90 (m, 2H), 6.01-6.20 (m, 2H), 5.75 (s br, 1H), 5.74 (d, 1H, *J* = 15.2 Hz), 4.43(d, 2H, *J* = 5.6 Hz), 3.78 (s, 3H), 2.13 (dd, 2H, *J=* 6.8 Hz, *J=* 13.6 Hz), 1.18-1.34 (m, 10H), 0.83-0.91 (m, 3H). ¹³C NMR (CDCl₃) δ (ppm): 14.08, 22.63, 28.65, 28.79, 29.10, 29.14, 31.78, 32.97, 43.20, 55.31, 100.87, 114.10, 121.36, 128.17, 129.26, 130.45, 141.77, 143, 57, 159.06, 166.14.

### Example 8: (2E,4E)-N-(3-methoxybenzyl)dodeca-2,4-diene amide (8)

White solid. Mp 82.2-84.2°C. HRMS (ESI) calcd for C₂₀H₂₉NO₂ (M + Na⁺) *m*/*z* 388.2090 found 388.2090. ¹H NMR (CDCl₃) δ (ppm): 7.22-7.30 (m, 2H), 7.18 (dd, 1H, *J=* 9.9 Hz, *J* = 15.0 Hz), 6.90 (dt, 1H, *J* = 1.0 Hz, *J* = 7.5 Hz), 6.86 (d, 1H, *J* = 8.4 Hz), 6.00-6.19 (m, 2H), 5.9 (s br 1H), 5.73(d, 1H, *J* = 15.0 Hz), 4.51 (d, 2H, *J* = 5.8 Hz), 3.85 (s, 3H), 2.12 (dd, 2H, *J* = 6.8 Hz, *J =* 13.3 Hz), 1.18-1.33 (m, 10H), 0.83-0.91 (m, 3H). ¹³C NMR (CDCl₃) δ (ppm): 14.06, 22.61, 28.77, 29.08, 29.12, 31.76, 32.95, 43.65, 55.22, 113.00, 113.37, 120,07, 121.27, 128.15, 129.71, 139.93, 141.86, 143.63, 159.88, 166.22.

### Example 9: (2E,4E)-N-(3-methoxyphenethyl)dodeca-2,4-diene amide (9)

White solid. Mp 95.3-96.5°C. HRMS (ESI) calcd for C₂₁H₃₁NO₂ (M + Na⁺) *m*/*z* 352.2247 found 352.2246. ¹H NMR (CDCl₃) δ (ppm): 7.14-7.24 (m, 2H), 6.75-6.80 (m, 2H), 6.73-6.75 (m, 1H), 6.05-6.10 (m, 2H), 5.67 (d, 1H, *J=* 14.9 Hz), 5.48 (s br 1H), 3.79(s, 3H), 3.59 (dd, 2H, *J* = 6.8 Hz, *J* = 13.0 Hz), 2.82 (t, 2H, *J* = 6.9 Hz), 2.13 (dd, 2H, *J* = 6.7 Hz, *J* = 13.0 Hz), 1.22-1.33 (m, 10H), 0.84-0.90 (m, 3H). ¹³C NMR (CDCl₃) δ (ppm): 14.06, 22.61, 28.77, 29.08, 29.11, 31.75, 32.94, 35.74, 40.53, 55.16, 111.90, 114.41, 121.08, 121.50, 128.14, 129.61, 140.57, 141.45, 143.40, 159.82, 166.31.

### Example 10: (2E,4E)-N-(biphenyl-4-yl)dodeca-2,4-diene amide (10)

Brownish solid. Mp 169.2-169.7°C. HRMS (ESI) calcd for C₂₄H₂₉NO (M + H⁺) *m*/*z* 348.2322 found 348.2323. ¹H NMR (CDC₃) δ (ppm): 7.61-7.69 (m, 2H), 7.53-7.60 (m, 4H), 5.42 (t, 2H, *J* = 7.8 Hz), 7.29-7.38 (m, 2H), 6.10-6.21 (m, 2H), 5.91 (d, 1H, *J* = 14.8 Hz), 2.17 (dd, 2H, *J* = 6.6 Hz, *J =* 13.4 Hz), 1.56 (s br 1H), 1.21-1.35 (m, 8H), 0.85-0.92 (m, 3H). ¹³C NMR (CDC₃) δ (ppm): 14.07, 22.62, 28.76, 29.08, 29.15, 31.76, 33.04, 120.24 (2C), 121.68, 126.78 (2C), 127.05, 127.56 (2C), 128.12, 128.74 (2C), 137.01, 137.46, 140.47, 142.95, 144.50, 164.61.

### Compound A: (2E,4E)-N-isobutyldodeca-2,4-diene amide

White solid. Mp 89.1-89.9°C. HRMS (ESI) calcd for C₁₆H₂₉NO (M + H⁺) *m*/*z* 252.2322 found 252.2322. ¹H NMR (CDCl₃) δ (ppm): 7.18 (dd, 1H, *J=* 9.7 Hz, *J=* 14.9 Hz), 6.01-6.17 (m, 1H), 5.74 (d, 4H, *J* = 14.7 Hz), 5.43 (s br, 1H), 3.16 (t, 2H, *J=* 6.53 Hz), 2.14 (dd, 2H, *J=* 7.1 Hz, *J=* 13.8 Hz), 1.79 (quintet, 1H, *J=* 6.8 Hz), 1.35-1.46 (m, 2H), 1.20-1.35 (m, 8H), 0.92 (d, 6H, *J =* 6.6 Hz), 0.88 (t, 3H, *J* = 7.1 Hz). ¹³C NMR (CDCl₃) δ (ppm): 14.04, 20.10 (2C), 22.59, 28.60, 28.78, 29.07, 29.10, 31.74, 32.92, 46.90, 121.78, 128.19, 141.19, 143.09, 166.40.

### Compound B: (2E,4E)-N-(2-methylbutyl)dodeca-2,4-diene amide

White solid. 1:1 enantiomeric mixture. Mp 67.1-68.1°C. HRMS (ESI) calcd for C₁₇H₃₁NO (M + H⁺) *m*/*z* 266.2478 found 266.2479. ¹H NMR (CDCl₃) δ (ppm): 7.17 (dd, 1H, *J=* 10.0 Hz, *J* = 14.9 Hz), 6.01-6.13 (m, 2H), 5.75 (d, 1H, *J =* 15.2 Hz), 5.55 (s, br 1H), 3.22-3.31 (m, 1H), 3.09-3.18 (m, 1H), 2.13 (dd, 2H, *J=* 6.8 Hz, *J=* 13.8 Hz), 1.51-1.62 (m, 1H), 1.35-1.47 (m, 3H), 1.21-1.33 (m, 8H), 1.01-1.21 (m, 1H), 0.83-0.95 (m, 9H). ¹³C NMR (CDCl₃) δ (ppm): 11.26, 14.07, 17.17, 22.62, 27.00, 28.81, 29.09, 29.12, 31.76, 32.94, 35.04, 45.18, 121.74, 128.19, 141.25, 143.18, 166.42.

### Compound C: (2E,4E)-N-isopentyldodeca-2,4-diene amide

White solid. Mp 72.1-73.4°C. HRMS (ESI) calcd for C₁₇H₃₁NO (M + H⁺) *m*/*z* 266.2478 found 266.2479. ¹H NMR (CDCl₃) δ (ppm): 7.17 (dd, 1H, *J* = 9.9 Hz, *J* = 15.0 Hz), 6.00-6.17 (m, 2H), 5.72 (d, 1H, *J=* 15.0 Hz), 5.49 (s, br 1H), 3.3-3.37 (m, 2H), 2.12 (dd, 2H, *J=* 6.8 Hz, *J* = 13.5 Hz), 1.62 (septet, 1H, *J* = 6.7 Hz), 1.35-1.47 (m, 4H), 1.18-1.33 (m, 8H), 0.91 (d, 6H, *J =* 6.4 Hz), 0.87 (t, 3H, *J =* 7.0 Hz). ¹³C NMR (CDCl₃) δ (ppm): 14.06, 22.44 (2C), 22.62, 25.85, 28.78, 29.08, 29.12, 31.76, 32.94, 37.89, 38.57, 121.67, 128.19, 141.19, 143.16, 166.31.

### Compound D: (2E,4E)-1-(pyrrolidin-1-yl)dodeca-2,4-dien-1-one

Yellowish solid. Mp 44.5-46.7°C. HRMS (ESI) calcd for C₁₆H₂₇NO (M + H⁺) *m*/*z* 250.2165 found 250.2165. ¹H NMR (CDCl₃) δ (ppm): 7.18-7.24 (m, 1H), 6.09-6.18 (m, 1H), 6.04-6.07 (m, 1H), 5.98-6.04 (m, 1H), 3.43-3.53 (m, 4H), 2.10 (dd, 2H, *J =* 7.1 Hz, *J=* 14.2 Hz), 1.87-1.96 (m, 2H), 1.77-1.86 (m, 2H), 1.31-1.42 (m, 2H), 1.16-1.30 (m, 8H), 0.80-0.87 (m, 3H). ¹³C NMR (CDCl₃) δ (ppm): 14.04, 22.59, 24.32, 26.09, 28.77, 29.07, 29.10, 31.74, 32.94, 45.80, 46.40, 119.83, 128.66, 142.13, 143.08, 165.16.

### Compound E: (2E,4E)-1-(piperidin-1-yl)dodeca-2,4-dien-1-one

White solid. Mp 40.6-41.9°C. HRMS (ESI) calcd for C₁₇H₂₉NO (M + H⁺) *m*/*z* 264.2322 found 264.2322. ¹H NMR (CDCl₃) δ (ppm): 7.21 (dd, 1H, *J=* 10.6 Hz, *J=* 14.8 Hz), 6.23 (d, 1H, *J=* 14.7 Hz), 6.11-6.20 (m, 1H), 5.98-6.07 (m, 1H), 3.53 (d, 4H, *J=* 45.1 Hz), 2.12(dd, 2H, *J=* 7.1 Hz, *J=* 14.0 Hz), 1.59-1.67 (m, 2H), 1.34-1.45 (m, 2H), 1.19-1.33 (m, 8H), 0.83-0.90 (m, 3H). ¹³C NMR (CDCl₃) δ (ppm): 14.04, 22.60, 24.64, 25.58, 26.63, 28.80, 29.08, 29.10, 31.74, 32.90, 43.10, 46.81, 118.51, 128.78, 142.50, 142.75, 165.65.

### Common Intermediates:

### (2E,4E)-dodeca-2,4-dienoic acid (silver oxide method)

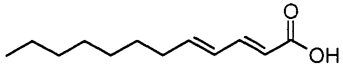

Silver oxide (1.93 g) was suspended in water (10 ml) and (2*E*,4*E*)-dodeca-2,4-dienal (1.00 g) was added. Aqueous sodium hydroxide (2.22 g, 50% solution) was added dropwise at such a rate that the temperature of the reaction mixture did not rise above 60°C. The reaction mixture was stirred at room temperature overnight and subsequently treated with 1N HCl, until the aqueous layer was acidic. The layers were separated and the aqueous layer was extracted with EtOAc. The combined organic layer was washed with brine, dried over Na₂SO₄, and the solvent was evaporated.

### Ethyl (2E,4E)-dodeca-2,4-dienoate

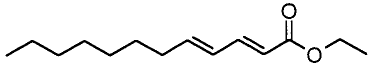

Sodium hydride (3.11 g) was suspended in dry tetrahydrofuran (200 ml) and cooled to 0°C. O,O-Diethyl ethoxycarbonylmethyl phosphonate (15.43 ml) was added dropwise. The reaction mixture was stirred at 0°C for 30 min and cooled to -78°C after which (*E*)-dec-2-enal (11.90 ml) was added dropwise. Stirring was continued at this temperature for 2 h, after which the mixture was warmed to room temperature. The reaction mixture was poured into water, and the aqueous phase was extracted with ethyl acetate. The combined organic layer was washed with brine, dried over sodium sulfate, and the solvent was evaporated. The crude product was purified by column chromatography.

### (2E,4E)-dodeca-2,4-dienoic acid (saponification)

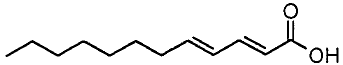

Ethyl (*2E*,*4E*)-dodeca-2,4-dienoate (11.84 g) was dissolved in methanol (130 ml) and 2M aqueous sodium hydroxide solution (35 ml) was added. The reaction mixture was heated to 60°C until full conversion was observed. After cooling down to room temperature the mixture was acidified with diluted aqueous HCl. The reaction mixture was extracted with ethyl acetate and the organic layer was washed with brine, dried over sodium sulfate, and solvents were evaporated. The crude product was used for the next step without further purification.

### (2E,4E)-dodeca-2,4-dienoyl chloride

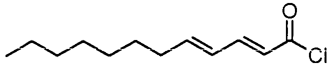

(*2E*,*4E*)-dodeca-2,4-dienoic acid (2.5 g) was dissolved in dichloromethane (25 ml). Oxalyl chloride (2.23 ml) was added and the reaction mixture was stirred at room temperature for 3 h. Solvent and excess oxalyl chloride are removed, and the residue was stripped several times with DCM. The crude acid chloride was used as such in next step.

### Synthesis of Example 12:

### Example 12:

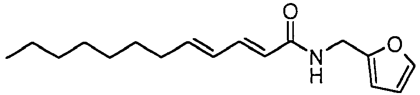

(*2E,4E*)-dodeca-2,4-dienoic acid (100 mg) was dissolved in dry *N,N*-dimethylformamide (2 ml). HATU (291 mg) and *N,N*-diisopropylethylamine (0.127 ml) were added and the reaction mixture was stirred for 30 min after which 2-aminomethyltetrahydrofuran (54 mg) was added. Reaction mixture was stirred at room temperature overnight. The reaction mixture was diluted with water, extracted with DCM, filtered over a phase separator and the organic layer was evaporated. The crude product was purified via flash column chromatography.

### Synthesis of Example 48:

### Intermediate 48a):

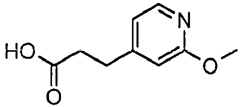

Ammonia was condensed into a round bottomed flask. While cooling the liquid ammonia with a CO₂/acetone bath, NaNH₂ (1.73 g) was added quickly. Then, 2-methoxy-4-methylpyridine (5.02 g) was added slowly via a syringe in the course of 5 min. The reaction mixture turned yellow immediately, but the starting material seemed to precipitate. The cooling bath was removed, and after 20 min, the reaction mixture had turned deep brown. The cooling bath was put in place again, and sodium 2-chloroacetate (4.74 g) was added portionwise in the course of 5 min. After stirring for 35 min, the cooling bath was removed; the reaction mixture had solidified partly. 50 min later, the reaction mixture was stirring again, and after another 40 min, NH₄Cl (3.29 g) was added carefully. The turbid, light brown reaction mixture immediately turned clear and yellow. The mixture was left stirring at RT overnight to allow the ammonia to evaporate. To the pasty residue, water (50 ml) was added to give a turbid, yellowish mixture. The pH of the mixture was adjusted to ∼4 using 3M aqueous HCl; at first, a white precipitate formed, followed by dark grey lumps. The mixture was extracted with DCM (2 x 50 mL), the combined extract was washed with brine (50 ml), dried over sodium sulfate, and concentrated *in vacuo.* The residue was taken up with 1M aqueous NaOH (50 ml), and washed with diethyl ether (50 ml). The basic aqueous layer was adjusted to pH ∼4-5 using 3M HCl (aq), and extracted with DCM (2 x 25 ml). Then, the pH was adjusted to pH ∼3-4, and the aqueous layer was extracted again with DCM (2 x 25 ml). The combined organic layer was dried over sodium sulfate, and concentrated *in vacuo.*

### Intermediate 48b):

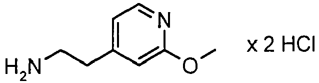

Sulfuric acid (9.78 g) was placed in a 50 ml round bottomed flask. 3-(2-Methoxypyridin-4-yl)propanoic acid (1.42 g) was added and the mixture was heated to 70°C. As the reaction mixture had become clear (within 1 h), sodium azide (417 mg) was added in very small portions in the course of 1.5 h. Stirring at 70°C was continued for 3.5 h; then, stirring was continued at RT overnight. The reaction mixture was poured on ice (∼80 g), and 5M aqueous NaOH (40 ml) was added to adjust pH =>10. The mixture was extracted with chloroform (50 ml and 2 x 25 ml). The combined organic layer was dried over sodium sulfate, concentrated *in vacuo* and stripped with DCM (50 ml). The crude product was dissolved in dry EtOH (5 ml); 4M HCl in dioxane (6 ml) was added to give a turbid, red mixture, and the solvents were evaporated. The residue was dissolved in EtOH (∼50 ml) by heating, under nitrogen atmosphere. The solution was allowed to cool for 3 h. Diisopropyl ether (∼2 ml) was added. After 1.5 h, a second portion of diisopropyl ether (∼2 ml) was added, and the solution was left standing at RT overnight. Additional diisopropyl ether (∼2 ml) was added and the solution was stored at 4°C for 3 d. The precipitate was filtered off, washed with diisopropyl ether and dried in a nitrogen stream.

### Example 48:

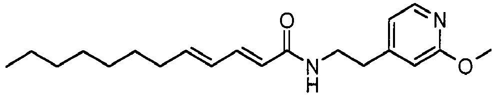

(*2E,4E*)-dodeca-2,4-dienoyl chloride (100 mg) was dissolved in DCM (2 ml). Intermediate 48b) (106 mg) was added followed by DMAP (56.9 mg). The reaction mixture was stirred at room temperature overnight and diluted with water. Phase separation was achieved using a phase separator. The organic layer was evaporated, and the crude product was purified via flash column chromatography.

### BIOLOGICAL ASSAYS

### A. Radioligand Displacement Assays on CB₁ and CB₂ Receptors

For the CB₁ receptor, binding experiments were performed in the presence of 0.39 nM of the radioligand [³H]CP55,940 (168 Ci/mmol) (Perkin Elmer, Waltham, MA, US) at 30°C in siliconized glass vials together with 7.16 µg of membrane recombinantly over-expressing CB₁ (No. RBHCB1M, Perkin Elmer, Waltham, MA, US), which was resuspended in 0.2 mL (final volume) binding-buffer (50 mM TRIS-HCl, 2.5 mM EGTA, 5 mM MgCl₂, 0.5 mg/mL fatty acid free BSA, pH 7.4). Test compounds were present at varying concentrations and the nonspecific binding of the radioligand was determined in the presence of 10 µM CP55,940 (Tocris Cookson Ltd., Bristol, UK). After 1.5 h incubation, the suspension was rapidly filtered through 0.05% polyethylenimine pre-soaked GF/C glass fiber filters on a 96-well cell harvester and washed nine times with 0.5 mL ice-cold washing-buffer (50 mM TRIS-HCl, 2.5 mM EGTA, 5 mM MgCl₂, 2% BSA, pH 7.4). Radioactivity on filters was measured with a Beckman LS 6500 scintillation counter in 3 mL Ultima Gold scintillation liquid (Perkin Elmer, Waltham, MA, US). Data collected from three independent experiments performed in triplicates were normalized between 100% and 0% specific binding for [³H]CP55,940. These data were graphically linearized by projecting Hill-plots, which allowed the calculation of IC₅₀ values. Derived from the dissociation constant (K*_{D}*) of [³H]CP55,940 and the concentration-dependent displacement (IC₅₀ value), inhibition constants (*Kᵢ*) of competitor compounds were calculated using the Cheng-Prusoff equation [*Kᵢ* = IC₅₀/(1+ L/K_{D})].

For CB₂-receptor binding studies 3.8 µg of membrane recombinantly over-expressing CB₂ (No. RBXCB2M, Perkin Elmer, Waltham, MA, US) were resuspended in 0.6 mL binding buffer (see above) together with 0.11 nM of the radioligand [³H]CP55,940. The CB₂-binding assay was conducted in the same manner as for CB₁.

### B. Critical micelle concentration (CMC) measurements

Dye micellization was carried out with a modified version of the method described by Eliyahu et al., Novel dextran-spermine conjugates as transfecting agents: comparing water-soluble and micellar polymers. Gene Ther. 2005 Mar;12(6):494-503. Compounds (from 2 mM stock solutions) were mixed at increasing concentrations with 0.1 nM fluorescein (free acid, 99%, Fluka, Switzerland) at room temperature in Nanopure distilled water. Experiments were carried out on 96-well microtiter plates (excitation at 485 nm, emission at 535 nm). Since the emission of fluorescein is pH-dependent, the pH of the mixture was kept constant at 6.9. The CMC range was determined as the concentration range where the first statistically significant increase in fluorescence was detected. At an appropriate resolution of the concentration range this increase was not gradual but sudden.

### C. Cellular uptake of [³H]-AEA

Human lymphoma U937 cells were grown in RPMI 1640 medium (Invitrogen, Basel, Switzerland) supplemented with 10% fetal bovine serum, 1 g/ml fungizone (amphotericin B), 100 units/ml penicillin, 100 g/ml streptomycin, and 2 mM L-glutamine (all from Invitrogen) and selected for the cellular AEA uptake assay. Human primary monocytes were isolated and cultured as described previously (Raduner S, Majewska A, Chen JZ, Xie XQ, Hamon J, Faller B, Altmann KH, Gertsch J. Alkylamides from Echinacea are a new class of cannabinomimetics. Cannabinoid type 2 receptor-dependent and -independent immunomodulatory effects. J Biol Chem. 2006 May 19;281(20):14192-206). 5x10⁵ cells were collected and suspended in 500 µL of sterile PBS (Sigma, San Louis, MO, US) using glass silanized tubes. Cells were pre-incubated 10 min at 37°C in presence of test compounds in a concentration range of 10⁻⁸ - 10⁻⁴ M or in presence of the same amount of solvent (control). Then a mixture of 50 nM [³H]-AEA (60 Ci/mmol) (American Radiolabeled Chemicals, Inc., San Louis, MO, US) and 50 nM of nonradioactive AEA (Tocris Cookson Ltd., Bristol, UK) was added and samples were incubated at 37°C for different times in the range of 1-60 min. The reaction was stopped by rapid filtration over Whatman GF/C filters pre-soaked in 0,25 % BSA. Filters were rinsed three times with 1 ml of ice-cold Krebs-HEPES buffer (118 mM NaCl, 4.7 mM KC1, 1.3 mM CaCl₂, 2.4 mM MgSO₄, 1 mM NaH₂PO₄, 20 mM NaHCO₃, 11.1 mM glucose, 3.98 µM Na₂EDTA, 110 mM ascorbic acid, 10 mM HepesNa and 1% BSA fatty acid free, pH 7.4) and 3 ml of Ultima Gold scintillation liquid (Perkin Elmer, Waltham, MA, US) was added. The filters-bound radioactivity was measured using a Beckman LS6500 scintillation counter. Results were expressed as % of AEA uptake versus cells treated with solvent and the EC₅₀ and IC₅₀ values were calculated from the sigmoidal curves, which were generated using the GraphPad Prism (GraphPad Software, San Diego, CA, US).

### D. Fatty acid amide hydrolase (FAAH) inhibition

FAAH activity was assessed using pig brain homogenate according to the method described by Omeir et al., Arachidonoyl ethanolamide-[1,2-14C] as a substrate for anandamide amidase. Life Sci. 1995;56(23-24):1999-2005, and adapted by Fowler et al., Ibuprofen inhibits rat brain deamidation of anandamide at pharmacologically relevant concentrations. Mode of inhibition and structure-activity relationship. J Pharmacol Exp Ther. 1997 Nov;283(2):729-34. Briefly, 10 µl of test compounds at the adequate concentration or solvent were incubated at 37°C for 20 min with 165 µl of diluted pig brain homogenate in 10 mM Tris-HCl and 1 mM EDTA, pH 7.6 (corresponding to 100 µg/ml of total protein), 1% w/v fatty acid free BSA (Sigma, San Louis, MO, US) and 25 µl of a mixture 1:1 of nonradioactive AEA [³H]-AEA (60 Ci mmol⁻¹) (concentration in the assay was 2 µM). Successively, 400 µl of a mixture 1:1 (v/v) of methanol:chloroform was added at each sample and, after vigorous vortexing, aqueous and organic phases were separated by centrifugation at 10000 rpm for 10 min. The radioactivity associated to the [³H]-ethanolamine (produced by FAAH-catalysed breakdown of [³H]-AEA) was measured upon addition of 3 ml of Ultima Gold scintillation liquid (Perkin Elmer, Waltham, MA, US) to 200 µl of the aqueous (upper) phase, using a Beckman LS6500 scintillation counter. Results were expressed as % of FAAH activity *vs.* pig brain homogenate treated only with solvent.

### E. Monoacylclycerol lipase (MAGL) inhibition

MAGL activity was evaluated using the Monoacylglycerol Lipase Inhibitor Screening Assay Kit (Cayman Chemicals, MI, US) and the experimental procedure was carried out according to the kit protocol. Briefly, 10 µl of test compounds at different concentrations (range 0,1-20 µM) were added to 150 µl of assay buffer, 10 µl of human recombinant MAGL and 10 µl of arachidonoyl-1-thio-glycerol (150 µM in the assay). The plate was incubated at room temperature for 5 min, afterward 10 µl of DTNB (5, 5'-dithiobis-(2-nitrobenzoic acid), Ellman's reagent) was added to each well. The plate was shaken for 10 seconds and the absorbance measured at 415 nm using Genius Pro spectrofluorimeter (Tecan, Grodig, Austria). Initial 100% enzymatic activty was calulated using solvent instead of test compounds, whilst background was measured without the human recombinant enzyme. The blank was subtracted from each value and results were expressed as % of initial enzymatic activity.

### Statistical analysis

Each experiment was carried out in triplicate and the results are expressed as mean values of at least three independent experiments ± standard error of the mean (SEM). The statistical significance of the data was evaluated by Student's *t*-test for unpaired data. P<0.05 was taken to be significant results.

The results obtained applying biological assays A to D are shown in Table 2 herein below.

**Table 2: Binding Affinity (Kᵢ) for Cannabinoid Receptors, Critical Micelle Concentrations (CMC), EC₅₀ Arachidonoylethanolamide Transport (AEAT) and Fatty Acid Amide Hydrolase (FAAH) Inhibition for Selected Examples of the Invention (Determination as Described in Biological Assays A to D)**

| | | | | | | |
|---|---|---|---|---|---|---|
| 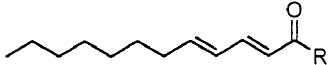 | | | | | | |

| Example | | Receptor affinity | | CMC | AEAT | FAAH inhibition at 1 µM |
|---|---|---|---|---|---|---|
| | R | Kᵢ CB₂ (nM) | Kᵢ CB₁ (nM) | (µM) | EC₅₀ (µM) | % of control |
| 1 | 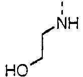 | >20'000 | >20'000 | >10 | 1.22 ± 0.24 | <10 |
| 9 | 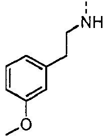 | 9440 ± 889 | >20'000 | >5 | 0.87 ± 0.12 | 14.63 ± 2.91 |
| 16 | 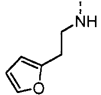 | n.d.²⁾ | n.d. | n.d. | 0.89 ± 0.40 | n.d. |
| 19 | 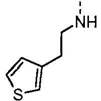 | n.d. | n.d. | n.d. | 0.52 ± 0.47 | n.d. |
| 20 | 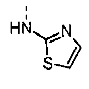 | n.d. | n.d. | n.d. | 0.78 ± 0.25 | n.d. |
| 28 | 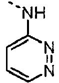 | n.d. | n.d. | n.d. | 10.8 ± 1.9 | n.d. |
| 36 | 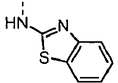 | n.d. | n.d. | n.d. | 0.56 ± 0.31 | n.d. |
| 42 | 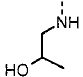 | n.d. | n.d. | n.d. | 3.9 ± 0.25 | n.d. |

| | | | | | | |
|---|---|---|---|---|---|---|
| ²⁾ n.d. = not determined | | | | | | |

For comparison, prior art compounds have also been profiled against the members of the endocannabinoid system. The results are depicted in Table 3.

**Table 3: Binding Affinity (Kᵢ) for Cannabinoid Receptors, Critical Micelle Concentrations (CMC), EC₅₀ Arachidonoylethanolamide Transport (AEAT) and Fatty Acid Amide Hydrolase (FAAH) Inhibition for Compounds Known from Literature (Determination as Described in Biological Assays A to D)**

| | | | | | | |
|---|---|---|---|---|---|---|
| 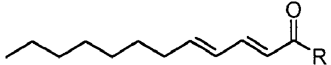 | | | | | | |

| | | Receptor affinity | | CMC | AEAT | FAAH inhibition at 1 µM |
|---|---|---|---|---|---|---|
| | R | Kᵢ CB₂ (nM) | Kᵢ CB₁ (nM) | (µM) | EC₅₀ (µM) | % of control |
| **A** | 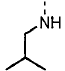 | 60 ± 7 | 1940 ± 213 | <0.5 | >10 | <10 |
| **B** | 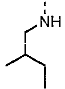 | 1104 ± 84 | 1512 ± 214 | >5 | >10 | <10 |
| **C** | 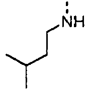 | 1455 ± 14 | >20'000 | >5 | >10 | <10 |
| **D** | 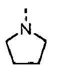 | <10'000 | >20'000 | >5 | >10 | <10 |
| **E** | 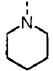 | 2639 ± 224 | 2192 ± 180 | >5 | >10 | <10 |

### F. Anandamide transport (human) cellular functional assay

Anandamide transport is determined using human U-937 cells as receptor source. [3H]-Anandamide (0.1 Ci/mmol), final concentration 0.1 µM is used as substrate. AM-404 is used as reference compound. Reactions are carried out in KRH buffer (pH 7.4) at 37°C for 20 min. The reaction is terminated by rapid vacuum filtration onto glass fiber filters. Radioactivity trapped onto filters is determined and compared to control values in order to ascertain any interactions of test compound with anandamide uptake (Hiiliard C.J. The movement of N-arachidonylethanolamide (anandamide) across cellular membranes. Chem. Physics Lipids. 2008;108:123-134, with modifications).

### G. Inhibiton of human recombinant Fatty acid amide hydrolase (fluoremetric method)

FAAH activity is measured using the modified method described by Omeir et al., Arachidonoyl ethanolamide-[1,2-14C] as a substrate for anandamide amidase. Life Sci. 1995;56(23-24):1999-2005. Human recombinant enzyme is preincubated with reference compound (methylarachidonlyfluorophosphonate, MAFP) and test compounds for 30 min at room temperature in 50 mM Tris containing 1 mM EDTA and 0.1% fatty acid free BSA (pH 8.0). Substrate (7-amino-4-methyl coumarin-arachidonamide, AMC-anandamide) is then added and incubated for 30 min at room temperature. The reaction is stopped by the addition of 2.5 µl of 10 µM URB597. Released 7-amino-4-methyl coumarin (AMC) is detected by fluorometry in order to ascertain any interactions of test compounds with the FAAH enzyme.

The results obtained applying biological assays F and G are shown in Table 4.

**Table 4: Inhibition of Anandamide Transport (AEAT) and Fatty Acid Amide Hydrolase (FAAH) for Selected Examples of the Invention (Determination as Described in Biological Assays F and G)**

| | | | |
|---|---|---|---|
| 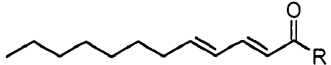 | | | |

| Example | | AEAT inhibition at 1 µM | FAAH inhibition at 1 µM |
|---|---|---|---|
| | R | % of control | % of control |
| 1 | 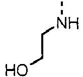 | 28 | 9 |
| 7 | 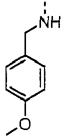 | 28 | 6 |
| 9 | 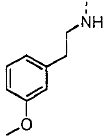 | 34 | 16 |
| 16 | 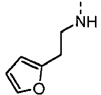 | 57 | 42 |
| 19 | 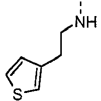 | 34 | 53 |
| 20 | 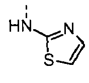 | 47 | -16 |
| 24 | 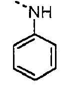 | 30 | 2 |
| 25 | 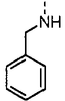 | 47 | 2 |
| 27 | 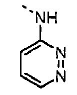 | 31 | -10 |
| 28 | 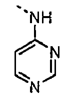 | 35 | -2 |
| 35 | 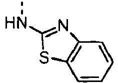 | 42 | 61 |
| 37 | 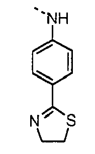 | 38 | 9 |
| 39 | 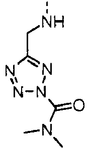 | 120 | 101 |
| 41 | 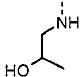 | 27 | 45 |
| 47 | 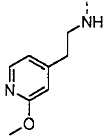 | 50 | 6 |
| 49 | 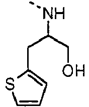 | 25 | 25 |
| 56 | 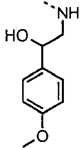 | 36 | -1 |
| 68 | 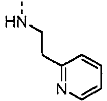 | 41 | -2 |
| 72 | 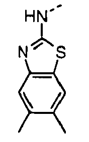 | 37 | 30 |
| 84 | 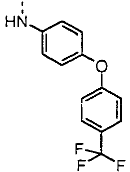 | 30 | 30 |

### H. In vivo model for pruritus associated with the oxazolone model of a delayed type hypersensitivity reaction

Scratching activity in mice is measured after topical application of the test compound. Ear thickness is measured and histology parameters are determined (see e.g. Elliott G.R. An automated method for registering and quantifying scratching activity in mice: use for drug evaluation. J. Pharmacol. Toxicol. Methods. 2000;44:453-459 and Gijbels M.J. Therapeutic interventions in mice with chronic proliferative dermatitis (cpdm/cpdm). Exp. Dermatol. 2000;9:351-358).

As evident from the results presented above, representative compounds of the present invention clearly exhibit a profile different from prior art compounds. Representative examples of the present invention have been shown to inhibit AEA re-uptake. In addition several examples also inhibit FAAH. In contrast, none of the prior art compounds inhibited AEA re-uptake or FAAH when tested in the assays described herein. Compound A exhibits a high affinity towards CB₂ and a moderate affinity towards CB₁, whereas compounds B and E are moderate affinity ligands for both, CB₁ and CB₂. Compound C shows a moderate affinity towards CB₂ while having no affinity towards CB₁ and finally, compound D does not interact with the endocannabinoid system at all. Therefore, the present invention discloses dodeca-*2E,4E*-diene amides with an unexpected profile regarding interaction with the endocannabinoid system.

### Examples of a Pharmaceutical Composition

### Composition Example 1:

| **Cream** | |
|---|---|
| Compound 1 | 1,00 |
| Cetostearyl alcohol | 7,00 |
| Macrogol-6-cetostearyl ether | 1,50 |
| Macrogol-25-cetostearyl ether | 1,50 |
| Liquid paraffin | 12,00 |
| Propylene glycol | 8,00 |
| Methylparaben | 0,15 |
| Ethylparaben | 0,08 |
| Butylhydroxytoluene | 0,04 |
| Disodium edetate | 0,05 |
| Water | 68,68 |

### Composition Example 2:

| **Gel** | |
|---|---|
| Compound 9 | 0,50 |
| Ethanol | 15,00 |
| Polyoxyl 40 Hydrogenated Castor Oil | 1,00 |
| Butylhydroxytoluene | 0,04 |
| Disodium edetate | 0,05 |
| Carbomer | 0,50 |
| Triethanolamine | 0,70 |
| Water | 82,21 |

### Industrial Applicability - Utility

The above results clearly show the improved technical effects provided by the dodeca-*2E,4E-*diene amide compounds of the present invention. It has surprisingly been found out by the present inventors that these compounds are suitable as active ingredients in pharmaceutical and cosmetic compositions, preferably dermatologic agents. Moreover, the compounds have shown to be effective in treating and/or preventing several diseases and conditions including, but not limited to inflammation, irritation, itching, pruritus, pain, oedema, and/or pro-allergic or allergic conditions, in particular when they are topically applied to the skin or mucosa in the form of pharmaceutical or cosmetic compositions containing a suitable carrier, preferably an apolar carrier. In particular, inflammation induced by cellular stress signals can effectively be attenuated by these compounds. Moreover, the compounds of the invention are effective in the treatment/prevention of hair growth conditions (e.g. alopecia, effluvium), sebaceous gland disorders (e.g. acne, seborrhea), benign and malignant skin tumors, hyperproliferative skin diseases (e.g. psoriasis), excessive hair growth (e.g. hirsutism), different forms of dermatitis, dry skin conditions and/or systemic sclerosis (e.g. scleroderma). The compounds of formula (1) are modulators of the endocannabinoid system and as such are useful in the treatment, control or prevention of the above mentioned diseases, disorders or conditions being responsive to one or more constituents of the endocannabinoid system including, but not limited to CB₁ and CB₂, TRPV1, GPR55, FAAH, monoacylglyceol lipase (MAGL) or AEAT. The pharmaceutical (dermatological) compositions and preparations containing the compounds of the present invention are highly active, even if the amount of active compound is reduced as compared to prior art compounds, and show less undesirable side-effects.

## Claims

1. A compound of the following formula (1) 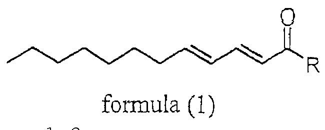 wherein R is a residue -NR¹R²
wherein R¹ is hydrogen and R² is selected from the group of hydroxyalkyl, arylalkyl, aryl, heteroarylalkyl and heteroaryl, with the proviso that, R² does neither comprise a -CH-C(=O)- moiety directly bound via the -CH group to the N atom of -NR¹R² nor a 12-membered unsaturated heterocyclic ring,
for use as a medicament, preferably as a dermatologic agent.

2. The compound for use according to claim 1 wherein
R² is selected from the group of substituted or unsubstituted C₆-C₂₀ aryl, substituted or unsubstituted C₅-C₂₀ heteroaryl, substituted or unsubstituted C₆-C₂₀ arylalkyl or heteroarylalkyl, or hydroxy -C₁-C₂₀-alkyl.

3. The compound for use according to claim 1 and/or claim 2 wherein the substituents are selected from halogens, preferably F, Cl and Br and I, C₁-C₁₀ alkyl, C₁-C₁₀ alkoxy, hydroxyl, -SH, -SO₃H, amine groups, -COOH, and COOR', wherein R' is a C₁-C₁₀ alkyl or an alkali metal, and preferably from F, Cl, C₁-C₆ alkyl, C₁-C₆ alkoxy, hydroxyl, -SH, -SO₃H, amine groups,-COOH, and COOR', wherein R' is a C₁-C₆ alkyl or an alkali metal.

4. The compound for use according to any one of claims 1 to 3 wherein
R² is selected from furanyl-C₁-C₁₀-alkyl, pyrrolyl-C₁-C₁₀-alkyl, (C₁-C₁₀-alkylpyrrolyl)-C₁-C₁₀-alkyl, benzyl, C₁-C₁₀-alkoxybenzyl, C₁-C₁₀-alkoxyphenalkyl, and biphenylyl.

5. The compound for use according to any one of claims 1 to 4 wherein
R² is selected from hydroxy-C₁-C₆-alkyl, furanylmethyl, (methylpyrrolyl)methyl, methoxybenzyl, methoxyphenethyl, and biphenylyl.

6. The compound for use according to any one of claims 1 to 5wherein
R² is selected from 2-hydroxyethyl, furan-3-ylmethyl, (1-methyl-1H-pyrrol-3-yl)methyl, 2-methoxybenzyl, 3-methoxybenzyl, 4-methoxybenzyl, 3-methoxyphenethyl, and biphenyl-4-yl.

7. The compound for use according to any one of claims 1 to 6 for use in treating and/or preventing inflammation, irritation, itching, pruritus, pain, oedema and/or pro-allergic or allergic conditions, preferably on the skin and/or mucosa of a mammal, and/or alopecia, effluvium, sebaceous gland disorders, benign and malignant skin tumors, hyperproliferative skin diseases, excessive hair growth, dermatitis, dry skin conditions and/or systemic sclerosis.

8. The compound for use according to any one of claims 1 to 7 which is applied topically on the skin or mucosa of a mammal.

9. Non-therapeutic use of the compound as defined in any one of claims 1-6 and 8 as a cosmetic, preferably for the cosmetic treatment of the skin and/or mucosa of a mammal.

10. Non-therapeutic use according to claim 9 for reducing irritation, itching, pruritus, and/or pro-allergic or allergic conditions, and alopecia, effluvium, sebaceous gland disorders, hyperproliferative skin diseases, excessive hair growth, dermatitis, dry skin conditions and/or systemic sclerosis.

11. Composition comprising the compound as defined in any one of claims 1-8 and a pharmaceutically and/or cosmetically acceptable carrier, preferably an apolar carrier, wherein the composition preferably is a liniment, skin cream, gel, or lotion.

12. A dodeca-*2E*,*4E*-diene amide of the following formula (1) 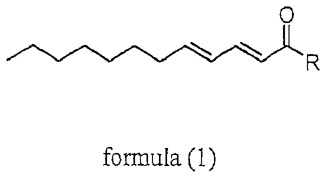 wherein R is a residue -NR¹R²
wherein R¹ is hydrogen and R² is selected from the group of hydroxyalkyl, arylalkyl, aryl, heteroarylalkyl and heteroaryl, provided that dodeca-*2E,4E*-diene p-hydroxyphenethyl amide is excluded, and/or R² does neither comprise a -CH-C(=O)- moiety directly bound via the -CH group to the N atom of -NR¹R² nor a 12-membered unsaturated heterocyclic ring.

13. The dodeca-*2E*, *4E*-diene amide according to claim 12 wherein
R² is selected from 2-hydroxyethyl, furan-3-ylmethyl, (1- methyl-1*H*-pyrrol-3-yl)methyl, 2-methoxybenzyl, 3-methoxybenzyl, 4-methoxybenzyl, 3- methoxyphenethyl, and biphenyl-4-yl.

## Patentansprüche

1. Verbindung der folgenden Formel (1) 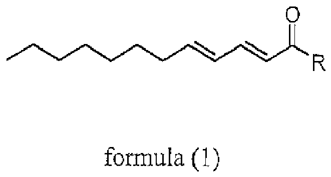 worin R einen Rest -NR¹R² darstellt,
worin R¹ Wasserstoff ist und R² ausgewählt ist aus der Gruppe aus Hydroxyalkyl, Arylalkyl, Aryl, Heteroarylalkyl und Heteroaryl, mit der Maßgabe, dass R² weder eine -CH-C(=O)- Einheit, die direkt über die -CH- Gruppe an das N-Atom von -NR¹R² gebunden ist, noch einen 12-gliedrigen ungesättigten heterozyklischen Ring umfasst,
zur Verwendung als Medikament, vorzugsweise als dermatologisches Mittel.

2. Verbindung zur Verwendung nach Anspruch 1, worin
R² ausgewählt ist aus der Gruppe aus substituiertem oder unsubstituiertem C₆-C-₂₀-Aryl, substituiertem oder unsubstituiertem C₅-C₂₀-Heteroraryl, substituiertem oder unsubstituiertem C₆-C₂₀-Arylalkyl oder Heteroarylalkyl oder Hydroxy-C₁-C₂₀-alkyl.

3. Verbindung zur Verwendung nach Anspruch 1 und/oder Anspruch 2, worin die Substituenten ausgewählt sind aus Halogenen, vorzugsweise F, Cl und Br und I, C₁-C₁₀-Alkyl, C₁-C₁₀-Alkoxy, Hydroxyl, -SH, -SO₃H, Amingruppen, -COOH und COOR', worin R' ein C₁-C₁₀-Alkyl oder ein Alkalimetal ist, und vorzugsweise aus F, Cl, C₁-C₆-Alkyl, C₁-C₆-Alkoxy, Hydroxyl, -SH, -SO₃H, Amingruppen, -COOH und COOR', worin R' ein C₁-C₆-Alkyl oder ein Alkalimetal ist.

4. Verbindung zur Verwendung nach einem der Ansprüche 1 bis 3, worin
R² ausgewählt ist aus Furanyl-C₁-C₁₀-alkyl, Pyrrolyl-C₁-C₁₀-alkyl, (C₁-C₁₀-Alkylpyrrolyl)-C₁-C₁₀-alkyl, Benzyl, C₁-C₁₀-Alkoxybenzyl, C₁-C₁₀-Alkoxyphenylalkyl und Biphenylyl.

5. Verbindung zur Verwendung nach einem der Ansprüche 1 bis 4, worin
R² ausgewählt ist aus Hydroxy-C₁-C₆-alkyl, Furanylmethyl, (Methylpyrrolyl)methyl, Methoxybenzyl, Methoxyphenethyl und Biphenylyl.

6. Verbindung zur Verwendung nach einem der Ansprüche 1 bis 5, worin
R² ausgewählt ist aus 2-Hydroxyethyl, Furan-3-ylmethyl, (1-Methyl-1*H*-pyrrol-3-yl)methyl, 2-Methoxybenzyl, 3-Methoxybenzyl, 4-Methoxybenzyl, 3-Methoxyphenethyl und Biphenyl-4-yl.

7. Verbindung zur Verwendung nach einem der Ansprüche 1 bis 6 zur Verwendung in der Behandlung und/oder Vorbeugung von Entzündungen, Irritationen, Juckreiz, Pruritus, Schmerz, Ödemen und/oder proallergischen oder allergischen Zuständen, vorzugsweise auf der Haut und/oder Schleimhaut eines Säugers, und/oder Alopecia, Effluvium, Talgdrüsenerkrankungen, gutartigen und bösartigen Hauttumoren, hyperproliferativen Hauterkrankungen, exzessivem Haarwachstum, Dermatitis, trockener Haut und/oder systemischer Sklerose.

8. Verbindung zur Verwendung nach einem der Ansprüche 1 bis 7, die auf die Haut oder Schleimhaut eines Säugers topisch aufgetragen wird.

9. Nichttherapeutische Verwendung der Verbindung, wie sie in einem der Ansprüche 1 bis 6 und 8 definiert ist, als Kosmetikum, vorzugsweise zur kosmetischen Behandlung der Haut und/oder Schleimhaut eines Säugers.

10. Nichttherapeutische Verwendung nach Anspruch 9 zur Linderung von Irritationen, Juckreiz, Pruritus, und/oder proallergischen oder allergischen Zuständen, und Alopecia, Effluvium, Talgdrüsenerkrankungen, hyperproliferativen Hauterkrankungen, exzessivem Haarwachstum, Dermatitis, trockener Haut und/oder systemischer Sklerose.

11. Zusammensetzung, umfassend die Verbindung, wie sie in einem der Ansprüche 1 bis 8 definiert ist, und einen phramazeutisch und/oder kosmetisch annehmbaren Träger, vorzugsweise einen apolaren Träger, worin die Zusammensetzung vorzugsweise ein Einreibemittel, eine Hautcreme, ein Gel oder eine Lotion darstellt.

12. Dodeca-*2E*,*4E*-dienamid der folgenden Formel (1) 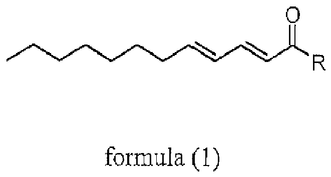 worin R ein Rest -NR¹R² ist,
worin R¹ Wasserstoff ist und R² ausgewählt ist aus der Gruppe aus Hydroxyalkyl, Arylalkyl, Aryl, Heteroarylalkyl und Heteroaryl, mit der Maßgabe, dass Dodeca-*2E,4E*-dien-p-hydroxyphenethylamide ausgeschlossen ist und/oder R² weder eine -CH-C(=0)- Einheit, die direkt über die -CH-Gruppe an das N-Atom von -NR¹R² gebunden ist, noch einen 12-gliedrigen ungesättigten heterozyklischen Ring umfasst.

13. Dodeca-*2E*,*4E*-dienamid nach Anspruch 12, worin
R² ausgewählt ist aus 2-Hydroxyethyl, Furan-3-ylmethyl, (1-Methyl-1*H*-pyrrol-3-yl)methyl, 2-Methoxybenzyl, 3-Methoxybenzyl, 4-Methoxybenzyl, 3-Methoxyphenethyl und Biphenyl-4-yl.

## Revendications

1. Un composé ayant la formule (1) suivante : 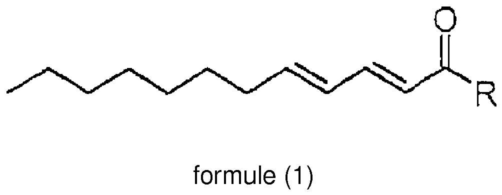 dans laquelle R représente un résidu -NR¹R² dans lequel R¹ représente l'hydrogène et R² est choisi au sein du groupe constitué par l'hydroxyalkyle, l'arylalkyle, l'aryle, l'hétéroarylalkyle et l'hétéroaryle, à condition que R² ne comprend ni un motif -CH-C(=O)- directement lié par le groupe -CH à l'atome N de -NR¹R², ni un anneau hétérocyclique non-saturé comprenant douze éléments,
pour l'utilisation en tant que médicament, préférablement comme un actif dermatologique

2. Le composé pour l'utilisation selon la revendication 1, dans lequel
R² est choisi au sein du groupe constitué par l'aryle substitué ou non-substitué en C₆ à C₂₀, l'hétéroaryle substitué ou non-substitué en C₅ à C₂₀, l'arylalkyle ou l'hétéroarylalkyle substitué ou non-substitué en C₆ à C₂₀, ou l'hydroxyalkyle en C₁ à C₂₀.

3. Le composé pour l'utilisation selon la revendication 1 et/ou la revendication 2, dans lequel
les substituants sont choisis parmi les halogènes, préférablement le F, le Cl et le Br et le I, l'alkyle en C₁ à C₁₀, l'alkoxy en C₁ à C₁₀, l'hydroxyle, le -SH, le -SO₃H, les groupes d'amines, le -COOH, et le COOR', R' étant un alkyle en C₁-C₁₀ ou un métal alcalin, et préférablement parmi le F, le Cl, l'alkyle en C₁ à C₆, l'alkoxy en C₁ à C₆, l'hydroxyle, le -SH, le -SO₃H, les groupes d'amines, le -COOH, et le COOR', R' étant un alkyle en C₁ à C₆ ou un métal alcalin.

4. Le composé pour l'utilisation selon l'une quelconque des revendications 1 à 3, dans lequel
R² est choisi parmi le furanylalkyle en C₁ à C₁₀, le pyrrolylalkyle en C₁ à C₁₀, (l'alkyle-en-C₁-C₁₀-pyrrolyl)-alkyle en C₁ à C₁₀, le benzyle, l'alkoxybenzyle en C₁ à C₁₀, l'alkoxyphénalkyle en C₁ à C₁₀ et le biphénylyle.

5. Le composé pour l'utilisation selon l'une quelconque des revendications 1 à 4, dans lequel
R² est choisi parmi l'hydroxyalkyle en C₁ à C₆, le furanylméthyle, le (méthylpyrrolyl)méthyle, le méthoxybenzyle, le méthoxyphénéthyle, et le biphénylyle.

6. Le composé pour l'utilisation selon l'une quelconque des revendications 1 à 5, dans lequel
R² est choisi parmi le 2-hydroxyéthyle, le furan-3-ylméthyle, (1-méthyl-1H-pyrrol-3-yl)méthyle, le 2-méthoxybenzyle, le 3-méthoxybenzyle, le 4-méthoxybenzyle, le 3-méthoxyphénéthyle, et le biphényl-4-yl.

7. Le composé pour l'utilisation selon l'une quelconque des revendications 1 à 6 pour l'utilisation dans le traitement et/ou la prévention d'inflammation, d'irritation, de démangeaison, de prurit, de douleur, d'oedème et/ou de conditions pro-allergiques ou allergiques, préférablement sur la peau et/ou la muqueuse d'un mammifère, et/ou d'alopécie, d'effluvium, de désordres des glandes sébacées, de tumeurs bénignes ou malignes, de maladies de peau hyperprolifératives, de croissance excessive de poils, de dermatite, de conditions de peau sèche et/ou de sclérose systémique.

8. Le composé pour l'utilisation selon l'une quelconque des revendications 1 à 7, qui est appliqué topiquement sur la peau ou la muqueuse d'un mammifère.

9. Utilisation non-thérapeutique du composé tel que défini dans l'une quelconque des revendications 1 à 6 et 8, en tant que produit cosmétique, préférablement pour le traitement cosmétique de la peau et/ou de la muqueuse d'un mammifère.

10. Utilisation non-thérapeutique selon la revendication 9 pour la réduction d'irritation, de démangeaison, de prurit et/ou de conditions pro-allergiques ou allergiques, et d'alopécie, d'effluvium, de désordres des glandes sébacées, de maladies de peau hyperprolifératives, de croissance excessive de poils, de dermatite, de conditions de peau sèche et/ou de sclérose systémique.

11. Composition comprenant le composé tel que défini dans l'une quelconque des revendications 1 à 8 et un excipient pharmaceutiquement et/ou cosmétiquement acceptable, préférablement un excipient non-polaire, dans lequel la composition est, préférablement, un liniment, une crème pour la peau, un gel, ou une lotion.

12. Un dodéca-2E,4E-diènamide ayant la formule (1) suivante : 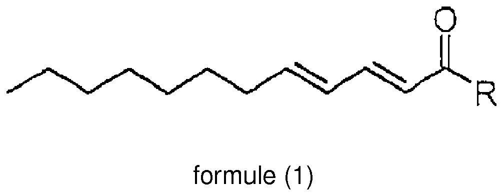 dans laquelle R est un résidu -NR¹R²
dans lequel R¹ représente l'hydrogène et R² est choisi parmi le groupe constitué par l'hydroxyalkyle, l'arylalkyle, l'aryle, l'hétéroarylalkyle et l'hétéroaryle, à condition que le dodéca-2E,4E-diènamide p-hydroxyphénéthylique soit exclu, et/ou que R² ne comprenne ni un motif -CH-C=O)- directement lié par le motif -CH à l'atome de N de -NR¹R², ni un anneau hétérocyclique non-saturé comprenant douze membres.

13. Le dodéca-2E,4E-diènamide selon la revendication 12, dans lequel R² est choisi parmi le 2-hydroxyéthyle, le furan-3-ylméthyle, le (1-méthyl-1 H-pyrrol-3-yl)méthyle, le 2-méthoxybenzyle, le 3-méthoxybenzyle, le 4-méthoxybenzyle, le 3-méthoxyphénéthyle, et le biphényle-4-yl.
